# EUROPEAN PATENT SPECIFICATION

(11) **EP 4 376 737 B1**
(45) Date of publication and mention of the grant of the patent: **08.07.2026**
(21) Application number: 22757222.9
(22) Date of filing: 21.07.2022
(51) Int. Cl.: A61B 17/42

(54) **HANDHELD GYNECOLOGICAL DEVICE, HANDLE AND ROD MEMBER**
TRAGBARE GYNÄKOLOGISCHE VORRICHTUNG, GRIFF UND STABELEMENT
DISPOSITIF GYNÉCOLOGIQUE PORTATIF, POIGNÉE ET ÉLÉMENT DE TIGE

(30) Priority: 29.07.2021 CH 0701132021
(43) Date of publication of application: 05.06.2024
(73) Proprietor: Aspivix SA, 1066 Epalinges (CH)
(72) Inventor: FINCI, Julien, 1095 Lutry (CH); TROMMER, Jérémie, 74800 Saint Piere en Faucigny (FR)
(74) Representative: Latscha Schöllhorn Partner AG
(86) International application number: PCT/EP2022/070485
(87) International publication number: WO 2023/006571

(56) References cited:
- WO-A1-2020/011616
- US-A1- 2017 196 593
- US-A1- 2017 281 231
- US-A1- 2018 078 749
- US-B1- 6 773 418
- US-B2- 10 751 088
- US-B2- 9 492 312
- ANONYMOUS: "Luer taper - Wikipedia", 25 June 2021 (2021-06-25), pages 1 - 2, XP055857510, Retrieved from the Internet <URL:https://en.wikipedia.org/wiki/Luer_taper> [retrieved on 20211103]

## Description

The use of medical instruments to assist during medical procedures and/or medical examinations is widespread. For instance, grasping devices are often used to handle and/or manipulate organs and/or body tissue of a patient.

In particular, many gynecological procedures and/or gynecological examinations, such as intra uterine contraception device (IUCD) insertion and removal, uterine tissue swabs for diagnostic purposes, cervix dilatation for uterine cavity curettage and/or for hysteroscopy, and measuring uterine cavity size during surgery, require an instrument to be inserted into the uterine cavity through the cervical canal.

In most anatomical cases, however, the cervical canal is typically angled with respect to the vaginal canal. Thus, prior to being able to perform the gynecological procedure, the cervical canal must be straightened in order to be able to insert an instrument into the uterine cavity.

Thus, for instance, grasping and manipulating the cervix of a patient is essential for performing many gynecological procedures and/or gynecological examinations, such as those described above.

Prior to performing the procedures, users open the vagina by means of a speculum in order to view the cervix of the patient. Then, the user inserts a grasping device in order to grasp the cervix and straighten the cervical canal to allow an instrument to be inserted into the uterine cavity.

One such grasping device is referred to as a tenaculum. Tenaculums typically have sharp-pointed hooks attached to a scissor-like handle. The cervix is grasped by the ends of the hooks by actuating the scissor-like handle to generate a clamping force. However, this form of grasping device can often be uncomfortable to the patient and can cause pain, bleeding and damage to the tissue of the cervix.

Thus, in recent years the use of vacuum-based grasping devices has been proposed in order to provide a gentler and more comfortable means for grasping the cervix of a patient.

For instance, WO 2020/011616 A1 discloses a grasping device comprising a suction head and a handle part connected to each other by a suction tube. The handle part comprises a vacuum pump with a vacuum chamber and a piston having a piston head movably arranged within the vacuum chamber. The vacuum pump further comprises a compressible spring and a button for releasing the compression of the spring. Once the suction head is properly placed, the user pushes the button to release the spring such that the piston is moved in the vacuum chamber, which generates a vacuum in the vacuum chamber to grip the cervix.

Moreover, WO 2020/074369 A1 discloses a gynecological device comprising a body part having a vacuum chamber and a rod member having a channel extending from a distal end to a proximal end. The gynecological device further comprises a cervix head arranged at the distal end of the rod member and a sealing mechanism configured to switch between an ambient state and a vacuum state. The sealing mechanism comprises a sleeve element which is axially movable relative to the vacuum chamber to switch the sealing mechanism between the ambient state and the vacuum state. The rod member is displaceable relative to the body member, thereby also axially displacing the sleeve element within the sleeve element to a first position to create a vacuum in the vacuum chamber. The sleeve element is moved further axially to a second position in order to transfer the vacuum from the vacuum chamber to the rod member and thus to the cervix head to grip the cervix.

Further, US 2017/196593 A1 shows a device for manipulating an uterus, US 10,751,088 B2 describes a gynecological apparatus, US 9,492,312 B2 shows an apparatus for inserting a device into a body cavity and WO 2020/011616 A1 describes a vacuum-based tenaculum. However, there remains a need for an improved comfort of use of vacuum-based gynecological grasping devices.

For instance, the prior art vacuum devices may not be suitable for the anatomy of all potential patients. Moreover, cleaning of prior art devices may be difficult, such that they are often designed to be discarded, which may increase the overall cost of the procedure. These issues have not, or at least not sufficiently, been addressed in the prior art.

It is therefore an object of the invention to provide a vacuum-based gynecological grasping device that can used for patients with different anatomies. It is a further object of the invention to provide a vacuum-based gynecological grasping device that is more economical to use.

This object is achieved by a handheld gynecological apparatus defined by the features of claim 1. Further developments and variations are defined by the features of the dependent claims.

Within the context of the present disclosure, the term "distal" and "distally" is to be understood as referring to a direction pointing away from a user of the device, i.e., a practitioner, and the term "proximal" and "proximally" is to be understood as referring to a direction pointing towards the user of the device during use of the device. Thus, the distal end of a component of the handheld gynecological apparatus is arranged further from the user than the proximal end of the component during use of the handheld gynecological apparatus. In other words, the distal end of a component of the handheld gynecological apparatus is introduced further into the patient than the proximal end of the component during use of the handheld gynecological apparatus.

The handheld gynecological apparatus comprises a body member having a proximal end and a distal end.

The body member may have a wall defining a vacuum chamber within the body member.

The vacuum chamber may be configured to receive or at least in part generate a vacuum therein during use of the handheld gynecological apparatus.

For instance, the vacuum chamber may be fluidically connected to a vacuum generating mechanism configured to generate a vacuum, such as a vacuum pump, which is preferably electrically and/or pneumatically driven.

The vacuum generating mechanism may alternatively be configured as a hand-driven pump, preferably comprising a piston which may be moved manually and/or via a spring force within the vacuum chamber to generate a vacuum therein.

The vacuum generating mechanism may be arranged at least partially within the vacuum chamber, or at least partially within the body member. Alternatively, the vacuum generating mechanism, preferably configured as an electrically and/or pneumatically powered vacuum pump, may be arranged outside of the body member, for instance on the floor or on a table in a clinical environment, and the vacuum generating mechanism may be fluidically connected to the vacuum chamber via a fluidical connecting element, such as a hose.

Preferably, the body member is made of a plastic material, which may at least partially be transparent. Preferably, the body member comprises at least one opening at its distal end and at least one opening at its proximal end, preferably formed in the wall of the body member.

Furthermore, the body member preferably has a substantially hollow cylindrical shape with a substantially annular-shaped cross-section. Alternatively, the body member may have a cross-sectional shape that is hollow and polygonal, such as a hollow shape with a quadrilateral or pentagonal cross-section. The hollow shape of the body member preferably at least partially forms a vacuum chamber, such as the vacuum chamber described above, within the body member.

The body member may be elongate, having a length preferably ranging from 6 cm to 20 cm, more preferably from 8 cm to 18 cm and having an outer diameter preferably less than 4 cm, more preferably less than 3 cm.

The body member may be of a one-piece construction. However, alternatively, the body member may be constructed of a plurality of different parts that are interconnected.

The body member may have a gripping feature, preferably arranged on an outer surface of the body member, to facilitate gripping the handheld gynecological apparatus at the body member during use.

For instance, the body member may comprise a handle attached to the outer surface of the body member and extending substantially radially or at least partially radially therefrom. For instance, the handle may extend from the body member with a radial and an axial directional component. Preferably, the handle extends at least partially circumferentially around at least a portion of the body member, preferably around the entire circumference of the body member.

Alternatively or additionally, the outer surface of the body member may have a roughened or gnarled portion to prevent the body member from slipping out of the hand of the user when gripping and maneuvering the handheld gynecological device. Alternatively or additionally, the outer surface of the body member may have at least one protrusion which protrudes from an outer surface of the body member which the user may grip during use.

The handheld gynecological device further comprises a rod member having a proximal end and a distal end. The rod member defines a lumen and is configured to be fluidically connected to the distal end of the body member.

The rod member may have a wall defining an outer surface and an inner surface, the inner surface preferably defining the lumen of the rod member. The rod member preferably has a substantially hollow cylindrical shape with a substantially annular-shaped cross-section. Alternatively, the rod member may have a cross-sectional shape that is hollow and polygonal, such as a hollow shape with a quadrilateral or pentagonal cross-section.

The rod member preferably has at least one opening at its proximal end and/or at least one opening at its distal end.

The rod member preferably has a total length ranging from 6 cm to 30 cm, more preferably from 10 cm to 25 cm, most preferably from 12 to 20 cm. Preferably, the rod member has an outer diameter of less than 3 cm, preferably less than 1.5 cm, more preferably of less than 1 cm.

Furthermore, the rod member preferably is substantially rigid to prevent or at least limit bending of the rod member when grasping and manipulating the cervix of a patient.

The rod member may be of a one-piece construction. However, alternatively, the rod member may be constructed of a plurality of different parts that are interconnected.

The rod member, more specifically the lumen defined therein, and the distal end of the body member, more specifically a vacuum chamber defined within the body member, may be permanently fluidically connected to each other, when the rod member and the body member are coupled to each other, i.e. at least during use of the handheld gynecological device.

Alternatively, the handheld gynecological device may comprise an actuating mechanism with an actuating member. The actuating member may be configured to be switched from a disconnected position, in which the body member, e.g., a vacuum chamber arranged within the body member, is fluidically disconnected from the lumen of the rod member, to a connected position, in which the body member, e.g., a vacuum chamber arranged within the body member, is fluidically connected to the lumen of the rod member.

Thus, a vacuum which is prevalent in the body member, e.g., in a vacuum chamber defined therein, may be transferred to the lumen of the rod member, and further to a suction head as described below, on demand, e.g., by actuating the actuating mechanism via the actuating member as described above. The vacuum may be generated in the body member, for instance, by a vacuum generating mechanism, such as a vacuum generating mechanism described above.

The actuating member is preferably coupled to the body member and/or the rod member. This preferably is achieved by movably mounting the actuating member, i.e., allowing rotational and/or translational movements thereof, to the body member and/or the rod member, for instance by configuring the body member and/or the rod member to receive at least a portion of the actuating member in at least a portion thereof. Alternatively or additionally, the actuating member may be configured such that a portion of the body member and/or the rod member may be received in at least a portion of the actuating member to couple the actuating member to the body member and/or the rod member. Different types of actuating members are described in Swiss Patent Application No. 01441/20 "Handheld gynecological device". These actuating members may also be employed in the apparatus of the present invention.

Alternatively, a connecting section, to which the actuating member is preferably releasably and/or movably attached, may be provided. The connecting section may be formed as a single unit with the body member or the rod member. Alternatively, the connecting section may be a separate section connected to the body member and/or the rod member.

The actuating member may comprise at least one sealing element configured and arranged to fluidically seal the body member from the lumen of the rod member when the actuating member is in the disconnected position (i.e., fluid tight). The at least one sealing element may further be configured and arranged to allow fluid connection between the lumen of the rod member and a vacuum chamber of the body member when the actuating member is in the connected position.

The sealing element may prevent or at least reduce air from entering the body member and/or the lumen of the rod member (in particular, at the proximal end of the rod member) from the environment when the actuating member is in the connected position to help maintain a vacuum.

The actuating member is preferably switched from the disconnected position to the connected position by displacing the actuating member relative to the body member and/or the rod member.

For instance, the actuating member may be displaced by rotating and/or translationally moving the actuating member relative to the body member and/or the rod member. For instance, the actuating member may be configured to be translationally moved in a direction substantially perpendicular to the longitudinal axis of the body member and/or the longitudinal axis of the rod member to switch the actuating member between the disconnected position and the connected position.

Alternatively, the actuating member may be configured to rotate about the longitudinal axis of the body member and/or about the longitudinal axis of the rod member to switch the actuating member between a disconnected position and a connected position.

Further alternatively, the actuating member may be configured to be moved translationally along the longitudinal axis of the body member and/or along the longitudinal axis of the rod member, i.e., in an axial direction, to switch the actuating member between a disconnected position and a connected position.

The handheld gynecological device further comprises a suction head having an engaging portion configured to engage at least a portion of a cervix of a patient. The suction head is fluidically connected to the distal end of the rod member, i.e., to the lumen of the rod member, and has a cross-section that is larger than a cross-section of the rod member.

Thus, the suction head may constitute the component of the handheld gynecological device which may physically engage a cervix of a patient to handle the cervix. For this reason, the suction head may be provided with various configurations, such as different geometries, materials and/or dimensions to adapt the suction head to the anatomy of the patient.

Prior to the gynecological and/or gynecological examination, the practitioner, i.e., the user of the handheld gynecological device, may select a suction head having a specific configuration suited, or at least more suited than suction heads having different configurations, for the patient's individual anatomy. The practitioner may then mount the selected suction head to the rod member.

Alternatively, the suction head may be integrally formed, e.g., integrally moulded, with the rod member. **In** this case, for example, the rod member may be replaceable.

The suction head may be substantially C-shaped, when viewed in the proximal direction (in particular, when viewed in a proximal direction along the longitudinal axis of the rod member). However, providing suction heads with different shapes, such as a circular shape or a D-shape, may also be feasible.

The suction head may comprise a wall, preferably extending around at least a portion of a circumference of the suction head and preferably defining a suction lumen. The wall may be concave with respect to the distal direction. Thus, the suction lumen is preferably open in the distal direction.

The wall of the suction head preferably defines an engaging portion configured to engage at least a portion of the cervix of a patient. Preferably, the suction head comprises a sealing element arranged on at least a portion of the engaging portion such that the sealing element may provide a substantially air-tight seal between the suction head and the portion of the cervix the handheld gynecological device is to be applied to.

Preferably, the position of the rod member along the longitudinal axis of the body member is fixed relative to the body member when the rod member is coupled to the body member. **In** other words, the rod member cannot be moved, or can only be minimally moved, relative to the body member along the longitudinal axis of the body member. For example, the rod member and the body member may be manufactured as a single integral part.

Alternatively or additionally, a separate connecting member may be arranged between the rod member and the body member which can provide a connection between the rod member and the body member. The rod member may be fixed relative to the body member such that also a rotational movement of the rod member relative to the body member is prevented (e.g., a rotational movement about the longitudinal axis of the rod member and/or about the longitudinal axis of the body member).

However, if desired, the rod member and the body member may also be movable with respect to each other, for instance, movable with respect to each other along the longitudinal axis of the body member and/or rotatable with respect to each other (e.g., rotatable about the longitudinal axis of the rod member and/or about the longitudinal axis of the body member).

The longitudinal axis of the body member and the longitudinal axis of the rod member may extend parallel to each other. Preferably, the longitudinal axis of the body member and the longitudinal axis of the rod member are coincident. In both cases, the actuating member can be displaced axially along the longitudinal axis of the rod member and the longitudinal axis of the body member.

However, it is also possible that the longitudinal axis of the body member and the longitudinal axis of the rod member extend at an angle to each other. In this case, the actuating member can be displaced axially along either the longitudinal axis of the rod member or the longitudinal axis of the body member.

The handheld gynecological device further comprises a vacuum generating mechanism configured to generate a vacuum within at least a portion of the handheld gynecological apparatus.

Preferably, the handheld gynecological device is configured such that the vacuum is generated in the body member and/or the rod member and/or the suction head. Preferably, the vacuum is generated in the body member and may be selectively transferred to the lumen of the rod member and the section head on demand, e.g., via actuation by the user, for instance, by actuating an actuating member, such as the actuating member described above.

The vacuum generating mechanism may be configured to be powered by an external source during use, such as electrically and/or pneumatically.

Alternatively, the vacuum generating mechanism may be configured to be powered by the user, such as by manually operating a moveable element, e.g., a piston housed within a vacuum chamber, to generate a vacuum by moving the moveable element.

The handheld gynecological device may be configured to be operated in at least two different modes, a first mode, in which the vacuum generating mechanism is configured to be powered by an external source, and a second mode, in which the vacuum generating mechanism is configured to be powered by the user, i.e., manually. The user may select which mode the gynecological device is to be operated in.

For this purpose, the handheld gynecological device may comprise at least two vacuum generating mechanisms, one of which may be configured to be powered by the user during use and a second of which may be configured to be powered by an external source during use.

Alternatively, a single vacuum generating mechanism may be provided and configured to be operated in both modes described above.

The vacuum generating mechanism may be integrated in the handheld gynecological device such that at least the body member, the rod member, the suction head and the vacuum generating mechanism may be moved, rotatably and/or translationally, as a single coherent unit as the handheld gynecological device is being maneuvered during use.

Alternatively, the vacuum generating mechanism may be a structurally independent and/or independently movable component during use, with the only connection between the vacuum generating mechanism and the body member and/or the rod member and/or the suction head being a fluidical connection, e.g., via a fluid connection hose.

**In** this case, the vacuum generating mechanism may be a stationary device, which may be arranged on a surface, such as a floor or a table top surface, during use.

The vacuum generating mechanism may be a mobile device, wherein the vacuum generating mechanism may be moved independently from the body member and/or the rod member and/or the suction head during use.

A first member and at least one second member of the handheld gynecological apparatus preferably are connectable to each other by a quick release coupling configured for manually coupling and uncoupling the first member and the second member to each other.

The first member preferably is the rod member and the second member preferably is one of the body member and the suction head.

By providing a quick release coupling configured for manually coupling and uncoupling the first member and the second member to each other, the rod member, the body member and/or the suction head may be removed, discarded, replaced, and/or interchanged prior to and/or after use of the handheld gynecological device by conveniently operating the quick release coupling to couple or uncouple the first member and the second member.

For instance, removing and discarding certain components of the handheld gynecological device, preferably the suction head and/or the rod member, after use of the handheld gynecological device may be desired. Since particularly the suction head and at least portions of the rod member may come into close contact with a patient's anatomy and/or with a patient's bodily fluids, particularly within the vagina of the patient, limiting the suction head and/or the rod member to a single use may be desired in order to increase hygienic standards, avoiding cross-contamination, and dispensing with or at least reducing the sterilisation efforts of the gynecological device after use.

Thus, the body member, and optionally also the rod member, may be reusable parts and the suction head, and optionally also the rod member, may be single-use parts.

This may allow the body member, and optionally also the rod member, to be configured as higher quality components, compared with the suction head and optionally also the rod member. This may allow the body member, and optionally also the rod member, to include features, such as higher quality materials, that would otherwise not be economical if the body member, and optionally also the rod member, were to be discarded after each use instead of being reusable.

Exchanging and/or interchanging different components of the gynecological device, preferably the suction head and/or the rod member, may also be desired prior to using the gynecological device in order to more precisely adapt the dimensional and/or geometric properties of the handheld gynecological device to the individual anatomical features of the patient on whom the gynecological procedure and/or gynecological examination is/are to be performed.

Since the anatomical features of humans can vary from person to person, adapting the dimensional and/or geometric properties of the handheld gynecological device to the anatomical features of the patient, on whom the gynecological procedure and/or gynecological examination is/are to be performed, may be particularly helpful to increase the safety and effectiveness of the gynecological device and enhance the comfort of the patient during the gynecological procedure and/or gynecological examination.

**In** particular, different suction heads, i.e., having different geometries and/or different dimensions and/or different materials, may be paired with a single, standardized body member.

While the prior art mentioned at the beginning has mentioned a general removability of certain components of the devices disclosed therein, the removing and replacing process to remove the components from the devices and replace them with different components is tedious, time-consuming and prone to errors and damage caused to the devices during the removing and replacing process.

A higher risk of making errors and/or causing damage to the devices during the removing and replacing process by the user may be particularly prevalent in high-stress situations, such as in clinical environments with the patient prior to and/or even during a gynecological procedure and/or gynecological examination.

The quick release coupling configured for manually coupling and uncoupling the first member and the second member to each other may provide a quick and efficient coupling and uncoupling mechanism for coupling and uncoupling the first member and the second member with each other on demand.

Moreover, the quick release coupling may reduce the risk of errors and damage caused to the devices during the removing and replacing process.

The quick release coupling may be configured to be manually operated to couple and uncouple the first member and the second member to each other by a human user in a nondestructive manner. Thus, the structural integrity of the first member and the second member may be substantially identical prior to and after coupling and/or uncoupling the first member and the second member via the quick release coupling.

The quick release coupling may be configured to couple and uncouple the first member and the second member to each other by a human user without the need and/or use of additional tools. Instead, the quick release coupling may be configured to couple and uncouple the first member and the second member to each other merely by the use of the hands of the user and by the forces generated by the user's body.

The quick release coupling may be configured to couple and uncouple the first member and the second member to each other a multiple amount of times, wherein a substantially identical degree of fluid-tightness between the first member and the second member is provided in each coupled state in which the first member is coupled to the second member.

This may ensure that a substantially constant functional operation of the gynecological device is provided when the first member and the second member are coupled and uncoupled repeatedly and/or when the first member and/or the second member is exchanged for a different first member and/or a different second member, respectively. For instance, one first member may be coupled to the second member and subsequently uncoupled from the second member and a different first member may subsequently be coupled to the second member and/or vice versa.

In each case, a substantially identical degree of fluid-tightness between the first member and the second member may be provided in each coupled state.

The quick release coupling may include elements for effecting the coupling between the first member and the second member, the elements being formed or at least provided on the first member and/or the second member.

Alternatively or additionally, an intermediate member may be arranged between the first member and the second member, at least in a coupled state. At least some of the elements for effecting the coupling between the first member and the second member may be formed or at least provided on the intermediate member. In this case, at least some of the elements for effecting the coupling between the first member and the second member may be formed or at least provided on the first member and/or the second member.

The gynecological device includes at least one alignment mark configured to indicate a starting orientation and/or a final orientation of at least one of the components of the handheld gynecological device relative to another component of the handheld gynecological device prior to and/or upon completion of the coupling of the first member with the second member via the quick release coupling. This may facilitate the coupling process of the first member with the second member via the quick release coupling.

The at least one first alignment mark is provided on the first member and at least one second alignment mark is provided on the second member. The first alignment mark may be aligned with the second alignment mark in a starting orientation and/or a final orientation of at least one of the components of the gynecological device relative to another component of the gynecological device prior to and/or upon completion of the coupling of the first member with the second member via the quick release coupling. The first and second alignment marks may be disaligned with respect to each other in the other one of the starting orientation and the final orientation.

The first and/or second alignment mark may be printed onto the first and second member, respectively. The first and/or second alignment mark may be formed as a recess or projection. Preferably, the quick release coupling is a luer slip coupling, a luer lock coupling, a bayonet coupling, a threaded coupling or a snap fit coupling.

Luer-type couplings, which include luer slip couplings and luer lock couplings, may provide a reliable, secure and substantially fluid-tight connection between the first member, i.e., the rod member, and the second member, i.e., the body member or the suction head.

Moreover, luer-type couplings may provide a quick and efficient coupling and uncoupling mechanism for coupling and uncoupling the first member and the second member with each other on demand.

Moreover, in many cases luer-type couplings have been standardized and/or widely examined and tested with respect to their dimensions and functional requirements, such as stability and/or leak-tightness.

Thus, luer-type couplings suitable for the desired application based on the demands of the desired application may be chosen from available standardized configurations. Luer-type couplings may be provided in accordance with ISO 80369-7:2021 as issued in May 2021.

In addition to the luer slip coupling or the luer lock coupling, the handheld gynecological device may further include a separate fixing mechanism configured to fix the first member to the second member. This may increase the secureness of the coupling between the first member and the second member.

The fixing mechanism may include at least one protrusion provided on one of the first member and the second member and at least one opening provided on the other of the first member and the second member, the opening being configured to receive the protrusion in a coupled state of the first member and the second member to prevent or at least limit movement of the first member and the second member relative to each other. The first member and the second member may both have such a protrusion and such an opening.

The protrusion may be flexible and/or deflectable, preferably at least towards a longitudinal axis of the first member and/or a longitudinal axis of the second member or away therefrom. The protrusion may be configured to snap into the opening in a coupled state of the first member and the second member.

A bayonet coupling may also provide a quick and efficient coupling and uncoupling mechanism for coupling and uncoupling the first member and the second member with each other on demand.

Moreover, bayonet couplings may provide a simple and space-efficient construction and may be constructed variably/flexibly according to the desired application.

A threaded coupling may further provide a quick and efficient coupling and uncoupling mechanism for coupling and uncoupling the first member and the second member with each other on demand.

Similar to luer-type couplings, threaded couplings have in many cases been standardized and/or widely examined and tested with respect to their dimensions and functional requirements, such as stability and/or leak-tightness.

Thus, the threaded coupling suitable for the desired application based on the demands of the desired application can be chosen from available standardized configurations.

The snap fit coupling may comprise one or more cantilevered arms, the one or more cantilevered arms preferably being formed or at least provided on the first member and/or the second member (e.g., integrally therewith).

The one or more cantilevered arms may alternatively be formed or at least provided on an intermediate member arranged between the first member and the second member, at least in a coupled state.

Preferably, a non-fixed end of the cantilevered arm has an engaging hook.

The cantilevered arm, with the engaging hook provided at the non-fixed end of the cantilevered arm, may be deflectable and engageable with an engaging section, the engaging section preferably being formed or at least provided on the first member, the second member and/or an intermediate member arranged between the first member and the second member, at least in a coupled state.

Thus, as the first member and the second member are being coupled to each other and/or uncoupled from each other, the cantilevered arm, with the engaging hook provided at the non-fixed end of the cantilevered arm, may be deflected and may subsequently engage with the engaging section to couple the first member to the second member in a fixed manner relative to each other in a fluid tight manner.

A bayonet coupling, snap fit couplings or threaded coupling may be used in combination with a luer-type connection, in particular in combination with a luer-slip connection.

Preferably, the luer slip coupling and the luer lock coupling each comprise a first tapered section arranged on the first member and a second tapered section arranged on the second member. The first tapered section and the second tapered section may be mated to each other such that the first tapered section and the second tapered section can be pressed together to couple the first member and the second member to each other. For some embodiments of the invention, the use of a tapered section on one of the first member and the second member may be sufficient.

By providing tapered sections on the first member and on the second member configured to be mated to each other, a relatively large mating surface may be provided, which may allow a secure and stably fixed connection between the first member and the second member. Moreover, this may provide a relatively large sealing surface to provide a substantially leaktight connection between the first member and the second member.

Moreover, the tapered sections can quickly and intuitively be mated, e.g., by inserting one tapered section into the other tapered section.

Preferably, the tapered sections extend about at least a section of a circumference, preferably about the entire circumference, of the first member and the second member, respectively.

Preferably, the tapered sections are rotationally symmetric with respect to a longitudinal axis of the first member and/or a longitudinal axis of the second member.

The tapered sections may be formed substantially conically.

The tapered sections may be tapered at a constant angle of taper. Preferably, the angle of taper is at least 4°, preferably at least 6°.

The tapered sections may be tapered in a distal or in a proximal direction, i.e. away from or towards the user.

One of the first tapered section and the second tapered section may be formed as a female connecting section and the other of the first tapered section and the second tapered section may be formed as a male connecting section. The male connecting section may be configured to be inserted into at least a section of the female connecting section as the first member and the second member are being coupled.

The first tapered section may be formed on an inwardly facing surface of the first member and the second tapered section may be formed on an outwardly facing surface of the second member.

Alternatively, the first tapered section may be formed on an outwardly facing surface of the first member and the second tapered section may be formed on an inwardly facing surface of the second member.

Preferably, the luer lock coupling additionally comprises at least one threaded section arranged on the first or the second member and at least one counter element arranged on the other of the first and the second member. The counter element may be configured to be screwed into the threaded section to couple the first member and the second member to each other.

The threaded section is preferably configured as a female threading. Alternatively, the threaded section may be configured as a male threading.

The counter element may be rotatable with respect to the first member or the second member, respectively, in order to engage the counter element with the threaded section and screw the counter element into the threaded section to provide a secure connection between the first member and the second member.

The counter element may be a protrusion, such as a tab, or a second threaded section.

Thus, in case a protrusion is provided as a counter element, the protrusion may be configured to rotatably engage at least a portion of the threaded section while coupling the first member and the second member. The protrusion may extend at least partially radially inwardly or outwardly from a surface of the first member or the second member, respectively, with respect to a longitudinal axis of the first member and/or a longitudinal axis of the second member, respectively.

Alternatively, instead of being a protrusion, the counter element may be a collar extending around at least a section of a circumference of the first member or the second member, respectively. Preferably, the collar extends along at least 5°, more preferably at least 10°, more preferably at least 15°, more preferably at least 20°, more preferably at least 25°, more preferably at least 30°, more preferably at least 35°, of the entire circumference of the first member or the second member, respectively.

Preferably, the collar may extend outwardly from a surface of the first member or the second member, respectively, i.e., away from the longitudinal axis thereof. Alternatively, the collar may extend inwardly from a surface of the first member or the second member, respectively, i.e., towards the longitudinal axis thereof.

In case the counter element is configured as a second threaded section, the second threaded section may be configured to rotatably engage at least a portion of the threaded section arranged on the other of the first member and the second member while coupling the first member to the second member.

Preferably, the luer lock coupling additionally comprises at least one threaded section arranged on the first or the second member and at least two counter elements are arranged on the other of the first and the second member.

The counter elements may each be a protrusion, such as a tab. The tabs may extend in a helical manner and/or may form a section of a threading.

Preferably, the counter elements are spaced apart from each other, and are preferably arranged diametrically opposite from each other on the first or the second member, respectively.

Providing such an arrangement may enable a secure coupled connection between the first member and the second member and may facilitate the coupling and uncoupling process.

Alternatively, the counter elements may each be a threaded section.

Preferably, the quick release coupling has at least one protrusion formed on the first member or the second member and the other of the first member and the second member has at least one engaging section. The protrusion and the engaging section may be configured such that the protrusion can pass the engaging section as the first member and the second member are being connected and such that the protrusion can engage the engaging section after the protrusion has passed the engaging section to lock the first member and second member in position relative to each other.

The engaging section may be configured to axially engage and/or abut, i.e., in the proximal and/or the distal direction, the protrusion. Thereby, movement of the protrusion with respect to the engaging section in the axial direction may be limited.

The engaging section may have at least one inwardly extending surface and/or outwardly extending surface, such as a collar.

Such an inwardly extending surface and/or outwardly extending surface may provide at least one retaining surface which may engage the protrusion and retain the first member and second member in position relative to each other or at least limit relative movement of the first member and the second member.

Alternatively, the engaging section may have a groove. The groove may provide at least one retaining surface which may engage the protrusion and retain the first member and second member in position relative to each other.

Preferably, the engaging section comprises at least one opening through which the protrusion can pass as the first member and the second member are being connected.

Preferably, the first member and/or the second member is/are configured to be rotated relative to each other once the protrusion has passed the engaging section such that the protrusion can engage the engaging section to lock the first member and the second member in position relative to each other.

The quick release coupling has at least one stop surface configured to limit rotation of the first member and/or the second member relative to each other as the first member and the second member are being coupled with each other.

Preferably, the stop surface is configured to limit rotation of the first member and/or the second member relative to each other about the longitudinal axis of the first member and/or the longitudinal axis of the second member as the first member and the second member are being coupled with each other.

Preferably, the quick release coupling has at least two stop surfaces configured to limit rotation of the first member and/or the second member relative to each other in a clockwise direction and/or a counter clockwise direction as the first member and the second member are being coupled with each other.

The stop surface(s) may be configured to limit rotation of the first member and/or the second member relative to each other in a clockwise direction and/or a counter clockwise direction about the longitudinal axis of the first member and/or the longitudinal axis of the second member to a rotational range of substantially 230° or less, preferably 210° or less, more preferably 190° or less, most preferably to 180°.

Preferably, the handheld gynecological apparatus comprises a plurality of protrusions. At least two protrusions may be arranged diametrically opposite from each other.

Preferably, the quick release coupling is configured to provide a fluid-tight seal between the first member and the second member.

Preferably, the first member is connectable to a third member of the gynecological apparatus by a second quick release coupling configured for manually coupling and uncoupling the first member and the third member to each other.

Preferably, the third member is the other one of the body member and the suction head.

Thus, the second member may be the body member and the third member may be the suction head. In this case, the rod member may be coupled to the body member via the first quick release coupling described above and the rod member may be coupled to the suction head via the second quick release coupling, which is further described below.

Alternatively, the second member may be the suction member and the third member may be the body member. In this case, the rod member may be coupled to the suction head via the first quick release coupling described above and the rod member may be coupled to the body member via the second quick release coupling.

By allowing a coupling and uncoupling via respective quick release couplings of at least three different components of the gynecological apparatus, i.e. the body member, the rod member and the suction head, an even more flexible adaption to the anatomy of the patient may be achieved.

As described with respect to the quick release coupling for coupling the first member with the second member, the second quick release coupling configured for manually coupling and uncoupling the first member and the third member to each other may provide a quick and efficient coupling and uncoupling mechanism for coupling and uncoupling the first member and the third member with each other on demand.

Moreover, the second quick release coupling may reduce the risk of errors and damage caused to the devices during the removing and replacing process.

The second quick release coupling may be configured to be manually operated to couple and uncouple the first member and the third member to each other by a human user in a nondestructive manner. Thus, the structural integrity of the first member and the third member may be substantially identical prior to and after coupling and/or uncoupling the first member and the third member via the second quick release coupling.

The second quick release coupling may be configured to couple and uncouple the first member and the third member to each other by a human user without the need and/or use of additional tools. Instead, the second quick release coupling may be configured to couple and uncouple the first member and the third member to each other merely by the use of the hands of the user and by the forces generated by the user's body.

The second quick release coupling may be configured to couple and uncouple the first member and the third member to each other a multiple amount of times, wherein a substantially identical degree of fluid-tightness between the first member and the third member is provided in each coupled state in which the first member is coupled to the third member.

This may ensure that a substantially constant functional operation of the gynecological device is provided when the first member and the third member are coupled and uncoupled repeatedly and/or when the first member and/or the third member is exchanged for a different first member and/or a different third member, respectively. For instance, one first member may be coupled to the third member and subsequently uncoupled from the third member and a different first member may subsequently be coupled to the third member and/or vice versa.

In each case, a substantially identical degree of fluid-tightness between the first member and the third member may be provided in each coupled state.

The second quick release coupling may include elements for effecting the coupling between the first member and the third member, the elements being formed or at least provided on the first member and/or the third member.

Alternatively or additionally, an intermediate member may be arranged between the first member and the third member, at least in a coupled state. At least some of the elements for effecting the coupling between the first member and the third member may be formed or at least provided on the intermediate member. In this case, at least some of the elements for effecting the coupling between the first member and the third member may be formed or at least provided on the first member and/or the third member.

The gynecological device may include a least one alignment mark configured to indicate a starting orientation and/or a final orientation of at least one of the components of the gynecological device relative to another component of the gynecological device prior to and/or upon completion of the coupling of the first member with the third member via the second quick release coupling. This may facilitate the coupling process of the first member with the third member via the second quick release coupling.

Preferably, at least one first alignment mark is provided on the first member and at least one second alignment mark is provided on the third member. The first alignment mark may be aligned with the second alignment mark in a starting orientation and/or a final orientation of at least one of the components of the gynecological device relative to another component of the gynecological device prior to and/or upon completion of the coupling of the first member with the third member via the second quick release coupling. The first and second alignment marks may be disaligned with respect to each other in the other one of the starting orientation and the a final orientation.

The first and/or second alignment mark may be printed onto the first and third member, respectively. The first and/or second alignment mark may be formed as a recess or projection.

Alternatively, the second quick release coupling may be omitted. Instead, the first member and the third member may be coupled to each other via a different coupling mechanism, such as a press fit and/or an adhesive connection between the first member and the third member. Further alternatively, the first member and the third member may be integrally formed, e.g., integrally moulded.

Preferably, the second quick release coupling is a luer slip coupling, a luer lock coupling, a bayonet coupling, a threaded coupling or a snap fit coupling.

As described above, luer-type couplings, which include luer slip couplings and luer lock couplings, may provide a reliable, secure and substantially fluid-tight connection between the first member, i.e., the rod member, and the third member, i.e., the body member or the suction head.

Moreover, luer-type couplings may provide a quick and efficient coupling and uncoupling mechanism for coupling and uncoupling the first member and the third member with each other on demand.

Moreover, in many cases luer-type couplings have been standardized and/or widely examined and tested with respect to their dimensions and functional requirements, such as stability and/or leak-tightness.

Thus, luer-type couplings suitable for the desired application based on the demands of the desired application may be chosen from available standardized configurations.

A bayonet coupling may also provide a quick and efficient coupling and uncoupling mechanism for coupling and uncoupling the first member and the third member with each other on demand. Moreover, bayonet couplings may provide a simple and space-efficient construction and may be constructed variably/flexibly according to the desired application.

A threaded coupling may further provide a quick and efficient coupling and uncoupling mechanism for coupling and uncoupling the first member and the third member with each other on demand.

Similar to luer-type couplings, threaded couplings have in many cases been standardized and/or widely examined and tested with respect to their dimensions and functional requirements, such as stability and/or leak-tightness. Thus, the threaded coupling suitable for the desired application based on the demands of the desired application can be chosen from available standardized configurations.

The snap fit coupling may comprise one or more cantilevered arms, the one or more cantilevered arms preferably being formed or at least provided on the first member and/or the third member.

The one or more cantilevered arms may alternatively be formed or at least provided on an intermediate member arranged between the first member and the third member, at least in a coupled state.

Preferably, a non-fixed end of the cantilevered arm has an engaging hook.

The cantilevered arm, with the engaging hook provided at the non-fixed end of the cantilevered arm, may be deflectable and engageable with an engaging section, the engaging section preferably being formed or at least provided on the first member, the third member and/or an intermediate member arranged between the first member and the third member, at least in a coupled state.

Thus, as the first member and the third member are being coupled to each other and/or uncoupled from each other, the cantilevered arm, with the engaging hook provided at the non-fixed end of the cantilevered arm, may be deflected and may subsequently engage with the engaging section to couple the first member to the third member in a fixed manner relative to each other.

Preferably, the luer slip coupling and the luer lock coupling each comprise a first tapered section arranged on the first member and a second tapered section arranged on the third member. The first tapered section and the second tapered section are mated to each other such that the first tapered section and the second tapered section can be pressed together to couple the first member and the third member to each other. For some embodiments of the invention, the use of a tapered section on one of the first member and the third member may be sufficient.

By providing tapered sections on the first member and on the third member configured to be mated to each other, a relatively large mating surface may be provided, which may allow a secure and stably fixed connection between the first member and the third member. Moreover, this may provide a relatively large sealing surface to provide a substantially leaktight connection between the first member and the third member.

Moreover, the tapered sections can quickly and intuitively be mated, e.g., by inserting one tapered section into the other tapered section.

Preferably, the tapered sections extend about at least a section of a circumference, preferably about the entire circumference, of the first member and the third member, respectively.

Preferably, the tapered sections are rotationally symmetric with respect to a longitudinal axis of the first member and/or a longitudinal axis of the third member.

The tapered sections may be formed substantially conically.

The tapered sections may be tapered at a constant angle of taper. Preferably, the angle of taper is at least 4°, preferably at least 6°.

The tapered sections may be tapered in a distal or in a proximal direction, i.e. away from or towards the user.

One of the first tapered section and the second tapered section may be formed as a female connecting section and the other of the first tapered section and the second tapered section may be formed as a male connecting section. The male connecting section may be configured to be inserted into at least a section of the female connecting section as the first member and the third member are being coupled.

The first tapered section may be formed on an inwardly facing surface of the first member and the second tapered section may be formed on an outwardly facing surface of the third member.

Alternatively, the first tapered section may be formed on an outwardly facing surface of the first member and the second tapered section may be formed on an inwardly facing surface of the third member.

Preferably, the luer lock coupling additionally comprises at least one threaded section arranged on the first or the third member and at least one counter element arranged on the other of the first and the third member, the counter element being configured to screw into the threaded section to couple the first member and the third member to each other.

The threaded section is preferably configured as a female threading. Alternatively, the threaded section may be configured as a male threading

The counter element may be rotatable with respect to the first member or the third member, respectively, in order to engage the counter element with the threaded section and screw the counter element into the threaded section to provide a secure connection between the first member and the third member.

The counter element may be a protrusion, such as a tab, or a second threaded section.

Thus, in case a protrusion is provided as a counter element, the protrusion may be configured to rotatably engage at least a portion of the threaded section while coupling the first member and the third member. The protrusion may extend at least partially radially inwardly or outwardly from the first member or the third member, respectively, with respect to a longitudinal axis of the first member and/or a longitudinal axis of the third member, respectively.

Alternatively, instead of being a protrusion, the counter element may be a collar extending around at least a section of a circumference of the first member or the third member, respectively. Preferably, the collar extends along at least 5°, more preferably at least 10°, more preferably at least 15°, more preferably at least 20°, more preferably at least 25°, more preferably at least 30°, more preferably at least 35°, of the entire circumference of the first member or the third member, respectively.

Preferably, the collar may extend outwardly from a surface of the first member or the third member, respectively, i.e., away from the longitudinal axis thereof. Alternatively, the collar may extend inwardly from a surface of the first member or the third member, respectively, i.e., towards the longitudinal axis thereof

In case the counter element is configured as a second threaded section, the second threaded section may be configured to rotatably engage at least a portion of the threaded section arranged on the other of the first member and the third member while coupling the first member to the third member.

Preferably, the luer lock coupling additionally comprises at least one threaded section arranged on the first or the third member and at least two counter elements are arranged on the other of the first and the third member.

The counter elements may each be a protrusion, such as a tab.

Preferably, the counter elements are spaced apart from each other, and are preferably arranged diametrically opposite from each other on the first or the third member, respectively.

Providing such an arrangement may enable a secure coupled connection between the first member and the third member and may facilitate the coupling and uncoupling process.

Alternatively, the counter elements may each be a threaded section.

Preferably, the second quick release coupling has at least one protrusion formed on the first member or the third member and the other of the first member and the third member has at least one engaging section. The protrusion and the engaging section may be configured such that the protrusion can pass the engaging section as the first member and the third member are being connected and such that the protrusion can engage the engaging section after the protrusion has passed the engaging section to lock the first member and third member in position relative to each other.

The engaging section may be configured to axially engage, i.e., in the proximal or the distal direction, the protrusion.

The engaging section may have at least one inwardly extending surface and/or outwardly extending surface, such as a collar.

Such an inwardly extending surface and/or outwardly extending surface may provide at least one retaining surface which may engage the protrusion and retain the first member and third member in position relative to each other or at least limit relative movement of the first member and the third member.

Alternatively, the engaging section may have a groove. The groove may provide at least one retaining surface which may engage the protrusion and retain the first member and third member in position relative to each other.

Preferably, the engaging section comprises at least one opening through which the protrusion can pass as the first member and the third member are being connected.

Preferably, the first member and/or the third member is/are configured to be rotated relative to each other once the protrusion has passed the respective engaging section such that the protrusion can engage the engaging section to lock the first member and the third member in position relative to each other.

Preferably, the first member and/or the third member is/are configured to be rotated relative to each other about the longitudinal axis of the first member and/or the longitudinal axis of the third member once the protrusion has passed the engaging section.

Preferably, the second quick release coupling has at least one stop surface configured to limit rotation of the first member and/or the third member relative to each other as the first member and the third member are being coupled to each other.

Preferably, the second quick release coupling has at least two stop surfaces to limit rotation of the first member and/or the third member relative to each other in a clockwise direction and/or a counter clockwise direction as the first member and the third member are being coupled to each other.

The stop surface(s) may be configured to limit the rotation of the first member and/or the third member relative to each other in a clockwise direction and/or a counter clockwise direction to a rotational range of substantially 230° or less, preferably 210° or less, more preferably 190° or less, most preferably to 180°.

Preferably, the handheld gynecological apparatus comprises a plurality of protrusions. At least two protrusions may be arranged diametrically opposite from each other.

Preferably, the second quick release coupling is configured to provide a fluid-tight seal between the first member and the third member.

Preferably, the vacuum generating mechanism is a vacuum pump driven by a drive mechanism.

The drive mechanism may be configured to effect a force on a vacuum generating element of the vacuum pump to generate a vacuum. For this purpose, the drive mechanism may have a force-effecting element, such as a spring, a pneumatic element or any other movable element capable of generating a force and transferring the force to the vacuum pump.

The drive mechanism can be incorporated in the handheld gynecological apparatus as a part thereof, for instance, at least partially within the body member and/or attached to the outside of the body member.

Alternatively, the drive mechanism may be arranged externally. **In** other words, the drive mechanism, e.g., an electrically and/or pneumatically driven vacuum pump, may not constitute a part of the handheld gynecological apparatus. **In** this case, the drive mechanism may be connectable to the handheld gynecological apparatus on demand.

The drive mechanism may be man-powered, e.g., by the user, such as by displacing a piston slidably arranged in a vacuum chamber to generate a vacuum in the vacuum chamber.

The drive mechanism may alternatively be powered pneumatically, e.g., via a compressed gas, and/or electrically, e.g., via a power outlet and/or a battery, preferably a rechargeable battery.

Preferably, the vacuum generating mechanism and a power source, such as at least one, preferably rechargeable, battery, for powering the vacuum generating mechanism may be arranged at least partially within the body member.

This may allow the handheld gynecological apparatus to be independent from cables, hoses etc., which may need to be connected to the handheld gynecological apparatus in order to provide external power to the vacuum generating mechanism and/or to provide a vacuum to the handheld gynecological apparatus.

Thus, the handheld gynecological apparatus can be maneuevered entirely free of such constraints. This may increase the flexibility and/or user-friendliness of the handheld gynecological apparatus.

Preferably, the vacuum generating mechanism is a pneumatically driven, electrically driven or manually driven vacuum pump.

Preferably, the vacuum generating mechanism is at least partially integrated or entirely within the body member. For example, the electrically driven vacuum pump may be at least partially or entirely be integrated into and/or enclosed by the body member.

Preferably, the handheld gynecological apparatus further comprises at least one filter arranged within the apparatus between the vacuum generating mechanism and the engaging portion of the suction head.

**In** particular, due to the vacuum generated in the handheld gynecological apparatus during its operation and use, bodily matters, such as fluids and/or solids, which stem from the patient's body may be suctioned into at least a section of the handheld gynecological apparatus.

By providing a filter within the apparatus between the vacuum generating mechanism and the engaging portion of the suction head, such bodily matters may be prevented from entering the vacuum generating mechanism or at least the amount of bodily matters which may enter the vacuum generating mechanism may be reduced while allowing gases, such as air, to pass the filter.

This may prevent or at least reduce the risk of contamination of the vacuum generating mechanism by bodily matters of the patient's body which may enter the handheld gynecological apparatus.

The filter may be arranged in the rod member, the suction head, or the body member.

Preferably, the handheld gynecological apparatus comprises a plurality of filters.

At least one filter may be arranged in the rod member and at least one filter may be arranged in the body member.

Preferably, the filter(s) may be anti-bacterial and/or hydrophobic.

Configuring the filter(s) to be anti-bacterial may prevent or at least inhibit bacterial contamination of the filter and/or of other components of the handheld gynecological apparatus and/or the spread of bacteria to sections of the handheld gynecological apparatus arranged proximally from the filter(s).

Hydrophobic filters are typically configured to repel liquids and resist wetting.

Thus, configuring the filter(s) to be hydrophobic may allow the filter(s) to repel liquids, such as bodily fluids stemming from the patient's body. This may further increase the filtering effect of the filter(s) with regard to bodily fluids stemming from the patient's body. Moreover, configuring the filter(s) to be hydrophobic may prevent the filter from being clogged by bodily fluids due to the water repelling features of the hydrophobic filter.

Preferably, the rod member is substantially rigid. Substantially rigid is to be understood as providing sufficient stiffness of the rod member such that the rod member does not bend or is otherwise deformed, or is only minimally bent or otherwise deformed, when manipulating the cervix.

Preferably, the longitudinal axis of the rod member and the longitudinal axis of the body member are parallel or coincide with each other.

Preferably, the rod member and/or the body member is/are substantially hollow cylindrical.

Preferably, the gynecological apparatus has a total length extending in the direction of the longitudinal axis of the rod member and wherein the total length is 60 cm or less, more preferably 50 cm or less, more preferably 40 cm or less.

Preferably, the rod member is 5 cm long or more, more preferably 10 cm long or more, or 15 cm or more. Preferably, the rod member is 30 cm long or less, preferably 25 cm long or less. For example, the length of the rod member may be about 19 cm. Such lengths are favourable for inserting the rod member through the vagina of the patient to engage with at least a portion of the cervix while allowing the user to operate the gynecological device, such as by actuating the actuating member.

Thus, depending on the required length of the gynecological device, for instance due to the varying anatomy of patients, the user can adapt the gynecological device according to the situation.

Preferably, the suction head defines a suction chamber fluidly connected to the lumen of the rod member, the suction chamber being delimited by a concave wall, with respect to a distal direction, and/or wherein an edge of the engaging portion of the suction head has a C shape. Such C shape is described in Applicant's earlier PCT publication number WO 2016/092458 A1.

Preferably, the edge forms a closed loop comprising at least a first edge portion and a second edge portion. Preferably, the first edge portion and the second edge portion do not lie in a common plane.

The following list of aspects provides alternative and/or further features of the present disclosure:
1. A gynecological apparatus, preferably configured to be a handheld gynecological apparatus, for cervix handling, comprising:
   - a body member having a proximal end and a distal end;
   - a rod member having a proximal end and a distal end, the rod member defining a lumen and being configured to be fluidly connected to the distal end of the body member;
   - a suction head having an engaging portion configured to engage at least a portion of a cervix of a patient, the suction head being fluidly connected to the distal end of the rod member, wherein preferably the suction head has a cross-section that is larger than a cross-section of the rod member; and

   wherein a first member and at least one second member of the gynecological apparatus are connectable to each other by a quick release coupling configured for manually coupling and uncoupling the first member and the second member to each other;
   wherein the first member is the rod member and the second member is one of the body member and the suction head.
2. The handheld gynecological apparatus according to aspect 1, further comprising a vacuum generating mechanism configured to generate a vacuum within at least a portion of the handheld gynecological apparatus.
3. The handheld gynecological apparatus according to aspect 1 or 2, wherein the quick release coupling is a luer slip coupling, a luer lock coupling, a bayonet coupling, a threaded coupling or a snap fit coupling.
4. The handheld gynecological apparatus according to aspect 3, wherein the luer slip coupling and the luer lock coupling each comprise a first tapered section arranged on the first member and a second tapered section arranged on the second member, wherein the first tapered section and the second tapered section are mated to each other such that the first tapered section and the second tapered section can be pressed together to couple the first member and the second member to each other.
5. The handheld gynecological apparatus according to aspect 3 or **4,** wherein the luer lock coupling comprises at least one threaded section arranged on the first or the second member and at least one counter element arranged on the other of the first and the second member, the counter element being configured to be screwed into the threaded section to couple the first member and the second member to each other.
6. The handheld gynecological apparatus according to any of aspects 1 to 3, wherein the quick release coupling has at least one protrusion formed on the first member or the second member and the other of the first member and the second member has at least one engaging section, wherein the protrusion and the engaging section are configured such that the protrusion can pass the engaging section as the first member and the second member are being connected and such that the protrusion can engage the engaging section after the protrusion has passed the engaging section to lock the first member and second member in position relative to each other.
7. The handheld gynecological apparatus according to aspect 6, wherein the engaging section comprises at least one opening through which the protrusion can pass as the first member and the second member are being connected.
8. The handheld gynecological apparatus according to aspect 6 or 7, wherein the first member and/or the second member is/are configured to be rotated relative to each other, preferably once the protrusion has passed the engaging section as the first member and the second member are being connected, such that the protrusion can engage the engaging section to lock the first member and the second member in position relative to each other.
9. The handheld gynecological apparatus according to aspect 8, wherein the quick release coupling has at least one stop surface configured to limit rotation of the first member and/or the second member relative to each other as the first member and the second member are being coupled with each other.
10. The handheld gynecological apparatus according to any of aspects 6 to 9, comprising a plurality of protrusions, preferably wherein at least two protrusions are arranged diametrically opposite from each other.
11. The handheld gynecological apparatus according to any of the preceding aspects, wherein the quick release coupling is configured to provide a substantially fluid-tight seal between the first member and the second member.
12. The handheld gynecological apparatus according to any of aspects 1 to 11, wherein the first member is connectable to a third member of the gynecological apparatus by a second quick release coupling configured for manually coupling and uncoupling the first member and the third member to each other.
13. The handheld gynecological apparatus according to aspect 12, wherein the third member is the other one of the body member and the suction head.
14. The handheld gynecological apparatus according to aspect 12 or 13, wherein the second quick release coupling is a luer slip coupling, a luer lock coupling, a bayonet coupling, a threaded coupling or a snap fit coupling.
15. The handheld gynecological apparatus according to aspect 14, wherein the luer slip coupling and the luer lock coupling each comprise a first tapered section arranged on the first member and a second tapered section arranged on the third member, wherein the first tapered section and the second tapered section are mated to each other such that the first tapered section and the second tapered section can be pressed together to couple the first member and the third member to each other.
16. The handheld gynecological apparatus according to aspect 14 or 15, wherein the luer lock coupling comprises at least one threaded section arranged on the first or the third member and at least one counter element arranged on the other of the first and the third member, the counter element being configured to screw into the threaded section to couple the first member and the third member to each other.
17. The handheld gynecological apparatus according to any of aspects 12 to **14,** wherein the second quick release coupling has at least one protrusion formed on the first member or the third member and the other of the first member and the third member has at least one engaging section, wherein the protrusion and the engaging section are configured such that the protrusion can pass the engaging section as the first member and the third member are being connected and such that the protrusion can engage the engaging section after the protrusion has passed the engaging section to lock the first member and third member in position relative to each other.
18. The handheld gynecological apparatus according to aspect 17, wherein the engaging section comprises at least one opening through which the protrusion can pass as the first member and the third member are being connected.
19. The handheld gynecological apparatus according to aspect 17 or 18, wherein the first member and/or the third member is/are configured to be rotated relative to each other, preferably once the protrusion has passed the respective engaging section as the first member and the third member are being connected, such that the protrusion can engage the engaging section to lock the first member and the third member in position relative to each other.
20. The handheld gynecological apparatus according to aspect 19, wherein the second quick release coupling has at least one stop surface configured to limit rotation of the first member and/or the third member relative to each other as the first member and the third member are being coupled to each other.
21. The handheld gynecological apparatus according to any of aspects 17 to 20, comprising a plurality of protrusions, preferably wherein at least two protrusions are arranged diametrically opposite from each other.
22. The handheld gynecological apparatus according to any of aspects 12 to 21, wherein the second quick release coupling is configured to provide a substantially fluid-tight seal between the first member and the third member.
23. The handheld gynecological apparatus according to any of the preceding aspects, wherein the vacuum generating mechanism is a vacuum pump driven by a drive mechanism.
24. The handheld gynecological apparatus according to any of the preceding aspects, wherein the vacuum generating mechanism is a pneumatically driven, electrically driven or manually driven vacuum pump.
25. The handheld gynecological apparatus according to any of the preceding aspects, wherein the vacuum generating mechanism is at least partially integrated within the body member.
26. The handheld gynecological apparatus according to any of the preceding aspects, further comprising at least one filter, the filter preferably being arranged within the apparatus between the vacuum generating mechanism and the engaging portion of the suction head.
27. The handheld gynecological apparatus according to aspect 26, wherein the filter is arranged in the rod member, the suction head, or the body member.
28. The handheld gynecological apparatus according to aspect 26 or 27, comprising a plurality of filters.
29. The handheld gynecological apparatus according to aspect 28, wherein at least one filter is arranged in the rod member and at least one filter is arranged in the body member.
30. The handheld gynecological apparatus according to any of aspects 26 to 29, wherein the filter/filters is/are anti-bacterial and/or hydrophobic.
31. The handheld gynecological apparatus according to any of the preceding aspects, wherein the rod member is substantially rigid.
32. The handheld gynecological apparatus according to any of the preceding aspects, wherein the longitudinal axis of the rod member and the longitudinal axis of the body member coincide with each other.
33. The handheld gynecological apparatus according to any of the preceding aspects, wherein the rod member and/or the body member are substantially hollow cylindrical.
34. The handheld gynecological apparatus according to any of the preceding aspects, wherein the gynecological apparatus has a total length extending in the direction of the longitudinal axis of the rod member and wherein the total length is 60 cm or less, more preferably 50 cm or less, more preferably 40 cm or less.
35. The handheld gynecological apparatus according to any of the preceding aspects, wherein the suction head defines a suction chamber fluidly connected to the lumen of the rod member, the suction chamber being delimited by a concave wall, with respect to a distal direction, and/or wherein an edge of the engaging portion of the suction head has a C shape.
36. The handheld gynecological apparatus according to aspect 35, wherein the edge forms a closed loop comprising at least a first edge portion and a second edge portion, wherein the first edge portion and the second edge portion do not lie in a common plane.
37. A handle for manipulating a handheld gynecological apparatus for cervix handling, the handle comprising a body member having a proximal end and a distal end, the distal end being configured to be connectable to a proximal end of a rod member of the handheld gynecological apparatus by a quick release coupling for manually coupling and uncoupling the body member and the rod member to each other.
38. The handle according to aspect 37, further comprising a vacuum generating mechanism configured to generate a vacuum within at least a portion of the body member, wherein the vacuum generating mechanism is at least partially integrated within the body member.
39. The handle according to aspect 38, wherein the vacuum generating mechanism is a vacuum pump driven by a drive mechanism.
40. The handle according to aspect 38 or 39, wherein the vacuum generating mechanism is a pneumatically driven, electrically driven, or manually driven vacuum pump.
41. A rod member for transferring a vacuum to a suction head of a handheld gynecological apparatus configured to engage at least a portion of a cervix of a patient, the rod member defining a lumen and having a proximal end and a distal end, wherein
   the distal end of the rod member is configured to be connectable to the suction head via a quick release coupling for manually coupling and uncoupling the rod member and the suction head to each other
      and/or
   the proximal end of the rod member is configured to be connectable to a distal end of a body member of the handheld gynecological apparatus via a quick release coupling for manually coupling and uncoupling the body member and the rod member to each other.

Embodiments of the present invention are further elucidated below with reference to the figures. The figures are schematic drawings and as such may not show all details of the systems and their components. Particularly, the drawings are not to scale and the shown dimensions are only exemplary and may vary. The drawings illustrate exemplary embodiments to provide a thorough understanding of the present invention. The drawings are not intended to limit the scope of the invention, which is defined by the appended claims.
- Fig. 1: shows a perspective view of a first embodiment of a handheld gynecological device according to the present invention, wherein the body member and the rod member are in an uncoupled state;
- Fig. 2: shows a perspective cross-sectional view of the embodiment of Fig. 1;
- Fig. 3: shows a further perspective view of the embodiment of Fig. 1;
- Fig. 4: shows an enlarged perspective view of the embodiment of Fig. 1;
- Fig. 5: shows a further enlarged perspective view of the embodiment of Fig. 1;
- Fig. 6: shows a perspective view of a second embodiment of a handheld gynecological device according to the present invention, wherein the body member and the rod member are in an uncoupled state;
- Fig. 7: shows a further perspective view of the embodiment of Fig. 6, wherein the body member and the rod member are assembled but not yet locked with each other;
- Fig. 8: shows a further perspective view of the embodiment of Fig. 6;
- Fig. 9: shows a further perspective view of the embodiment of Fig. 6, wherein the body member and the rod member are assembled but not yet locked with each other;
- Fig. 10: shows a perspective view of a third embodiment of a handheld gynecological device according to the present invention, wherein the suction head and the rod member are in an uncoupled state;
- Fig. **11**: shows a further perspective view of the embodiment of Fig. 10;
- Fig. 12: shows a further perspective view of the embodiment of Fig. 10, wherein the suction head and the rod member are in a coupled state;
- Fig. 13: shows a further perspective view of the embodiment of Fig. 10, wherein the suction head and the rod member are in a coupled state.

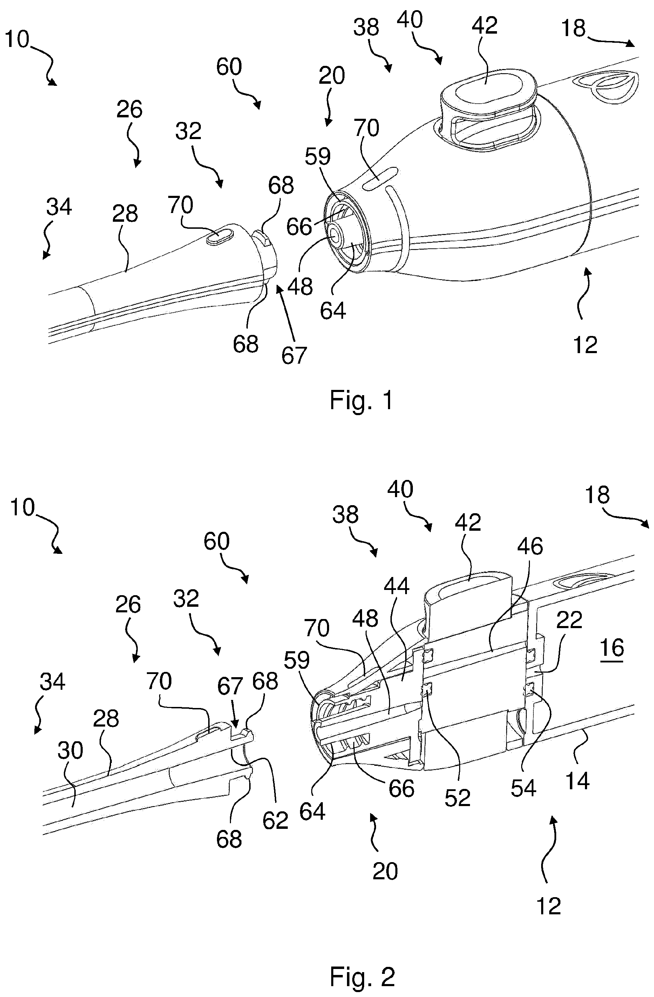

Figs. 1 to 5 show a handheld gynecological device 10 for cervix handling according to a first embodiment of the present invention.

The gynecological device 10 comprises a body member 12 having a wall 14 defining a vacuum chamber 16 and having a proximal end 18 and a distal end 20. The body member 12 further comprises an opening 22 at its distal end 20.

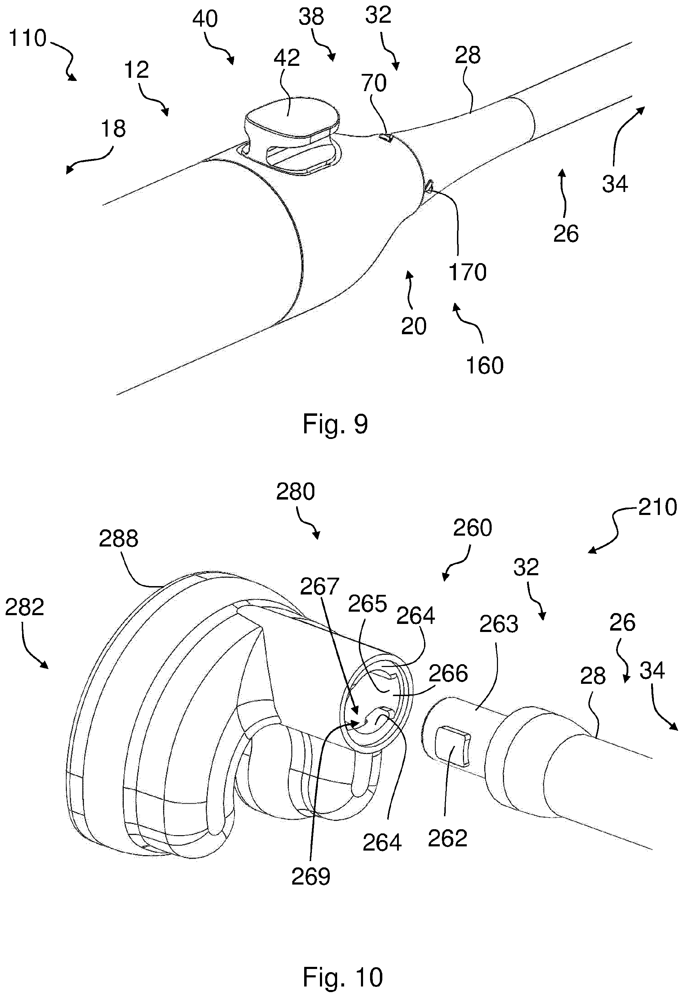

The handheld gynecological device 10 further comprises a rod member 26 having a wall 28 defining a lumen 30 and further having a proximal end 32 and a distal end 34 (not visible in Figs. 1 to 5; cf. Figs. 10 to 13).

A suction head (not shown in the embodiment of Figs. 1 to 5) having an engaging portion configured to engage at least a portion of a cervix of a patient may be fluidly connected to the distal end 34 of the rod member 26, and thus to the lumen 30 of the rod member 26. The suction head may have a cross-section that is larger than a cross-section of the rod member 26.

Such a suction head is shown in Figs. 10 to 13 and described further below with respect to a third embodiment of the handheld gynecological device, which is denoted with the reference sign 210 therein.

In the embodiment shown in Figs. 1 to 5, the longitudinal axis of the body member 12 is coincident with a longitudinal axis of the rod member 26. However, it is also feasible that the longitudinal axis of the body member 12 is not coincident with the longitudinal axis of the rod member 26. For instance, the longitudinal axis of the body member 12 may be non-coincident to the longitudinal axis of the rod member 26, but may extend parallel to the longitudinal axis of the rod member 26. Alternatively, the longitudinal axis of the body member 12 may extend at an angle to the longitudinal axis of the rod member 26.

The body member 12 comprises a connecting section 38 which structurally connects the rod member 26 to the body member 12.

As shown in the embodiment of Figs. 1 to 5, the connecting section 38 is a separate part which is fixedly connected to the section of the body member 12 proximal to the connecting section 38.

However, the connecting section 38 and the section of the body member 12 proximal to the connecting section 38 may alternatively be configured as a single integral unit, in other words as a one-piece component.

The gynecological device 10 further comprises an actuating mechanism 40 arranged at least partially within the connecting section 38, the actuating mechanism 40 having an at least partially moveable actuating member 42 and a static member 44. The actuating member 42 may be moveable relative to the static member 44.

The actuating mechanism 40 is shown in detail in Fig. 2.

As shown in Fig. 2, the actuating member 42 is slidably attached to the static member 44 such that the actuating member 42 can be slid partially within and along the static member 44, in a direction substantially perpendicular to the longitudinal axis of the body member 12 and/or the longitudinal axis of the rod member 26, to switch the actuating member 42 between a disconnected position and a connected position, as described further below. As shown in Fig. 2, the actuating member 42 is in the disconnected position.

Alternatively, the actuating member 42 may be configured to rotate about the longitudinal axis of the body member 12 and/or about the longitudinal axis of the rod member 26 to switch the actuating member 42 between the disconnected position and the connected position. Alternatively, the rotational movement of the actuating member 42 may be about an axis which does not coincide with the longitudinal axis of the body member 12 and/or the rod member 26, such as about an axis which extends at an angle to the longitudinal axis of the body member 12 and/or the rod member 26, preferably about an axis which is perpendicular to the longitudinal axis of the body member 12 and/or the longitudinal axis of the rod member 26.

Further alternatively, the actuating member 42 may be configured to be moved translationally along the longitudinal axis of the body member 12 and/or along the longitudinal axis of the rod member 26, i.e., in an axial direction.

The actuating member 42 comprises a channel 46 extending therethrough. As shown in Fig. 2, the channel 46 is tapered in the proximal direction, i.e., towards the user during use of the gynecological device 10. Alternatively, the channel 46 may be tapered in the opposite direction, i.e., in the distal direction. The channel 46 may also not be tapered at all.

The static member 44 comprises a channel 48 formed in a section thereof.

The handheld gynecological device 10 further comprises a first seal member 52 arranged in a section of the actuating member 42 and a second seal member 54 arranged partially around the opening 22 of the body member 12. The seal members 52, 54 may alternatively be arranged elsewhere.

The seal members 52, 54 may prevent ambient air from entering the channels 46, 48, the vacuum chamber 16 and/or the lumen 30. However, the sealing may also be achieved in a different manner, such as by using sealing coatings.

In the disconnected state, as shown in Fig. 2, the channel 46 is arranged offset relative to the channel 48 and to the opening 22. Thus, in the disconnected state, the vacuum chamber 16 and the lumen 30 of the rod member 26 are fluidly disconnected from each other.

Thus, a vacuum which may be generated in the vacuum chamber 16 of the body member 12, e.g., by a vacuum generating mechanism (not shown), is not transferred to the channel 46 of the actuating member 42, to the channel 48 of the static member 48 and to the lumen 30 of the rod member 26.

To switch the actuating member 42 form the disconnected position to the connected position, the actuating member 42 is displaced substantially perpendicular to the longitudinal axis of the body member 12 and/or the rod member 26. The channel 46 formed in the actuating member 42 is thereby also displaced substantially perpendicular to the longitudinal axis of the body member 12 and/or the rod member 26.

Thus, in the connected position, the channel 46 formed in the actuating member 42 fluidly connects the opening 22 of the body member 12 and the channel 48 formed in the static member 44.

Thus, in this connected state, a vacuum which may be generated in the vacuum chamber 16 of the body member 12, e.g., by a vacuum generating mechanism (not shown), may be transferred to the lumen 22 of the body member 18, to the channel 46 of the actuating member 42, to the channel 48 of the static member 44, to the lumen 30 of the rod member 26 and finally to a suction head.

The gynecological device 10 further comprises a quick release coupling 60 configured for manually coupling and uncoupling the rod member 26 and the body member 12.

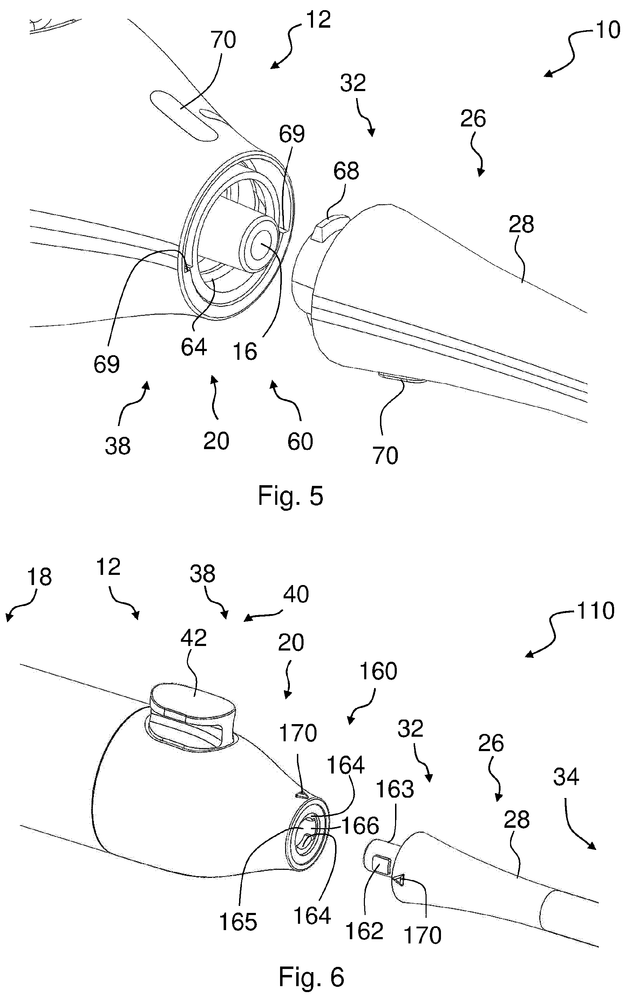

According to the embodiment shown in Figs. 1 to 5, the quick release coupling 60 is configured as a luer lock coupling. Alternatively, the quick release coupling 60 may be configured as a luer slip coupling, a bayonet coupling (see Figs. 6 to 9), a threaded coupling or a snap fit coupling.

The quick release coupling 60 in the embodiment shown in Figs. 1 to 5, i.e., configured as a luer lock coupling, comprises a first tapered section 62 arranged on an inwardly facing surface of the rod member 26 and a second tapered section 64 arranged on an outwardly facing surface of the connecting section 38 of the body member 12. The first tapered section 62 and the second tapered section 64 are mated to each other such that the first tapered section 62 and the second tapered section 64 can be pressed together to couple the rod member 26 and the body member 12 to each other in a fluid-tight manner.

In the embodiment shown in Figs. 1 to 5, the first tapered section 62 is configured as a female connecting member and the second tapered section 64 is configured as a male connecting member, wherein the male connecting member is received in the female connecting member as the body member 12 is being coupled with the rod member 26. A configuration in which the male connecting member is arranged on the rod member 28 and the female connecting member is arranged on the body member 12 is also feasible.

In the embodiment shown in Figs. 1 to 5, the first tapered section 62 and the second tapered section 64 are tapered in the distal direction (i.e. they decrease in diameter and/or width in the distal direction). However, the first tapered section 62 and the second tapered section 64 may also be tapered in the proximal direction.

The quick release coupling 60 in the embodiment shown in Figs. 1 to 5, i.e., configured as a luer lock coupling, additionally comprises a threaded section 66.

According to the first embodiment shown in Figs. 1 to 5, the threaded section 66 is arranged on an inner surface of a sleeve section 59 of the static member 44, the sleeve section 59 protruding distally from the static member 44 within the connecting section 38.

However, alternatively, the threaded section 66 may be arranged elsewhere, e.g., on any inwardly or outwardly facing surface of the body member 12.

In order to engage the threaded section 66 of the connecting section 38 of the body member 12, the rod member 26 comprises a hub 67 with two counter elements 68 arranged thereon. The counter elements 68 are configured to be screwed into the threaded section 66 to couple the rod member 26 and the body member 12 to each other.

Alternatively, it is also feasible to provide a single counter element 68 or more than two counter elements 68 to engage the threaded section 66.

The counter elements 68 may be arranged diametrically opposite from each other.

The counter elements 68 may extend from a surface of the rod member 26 with at least a radial directional component, with respect to the longitudinal axis of the rod member 26. The counter elements 68 may extend from the rod member 26 with at least a radial directional component, with respect to the longitudinal axis of the rod member 26, and an axial directional component.

The counter elements 68 may extend along a helical path.

According to the first embodiment shown in Figs. 1 to 5, the counter elements 68 extend radially away from the longitudinal axis of the rod member 26. However, the counter elements 68 may alternatively extend radially towards the longitudinal axis of the rod member 26.

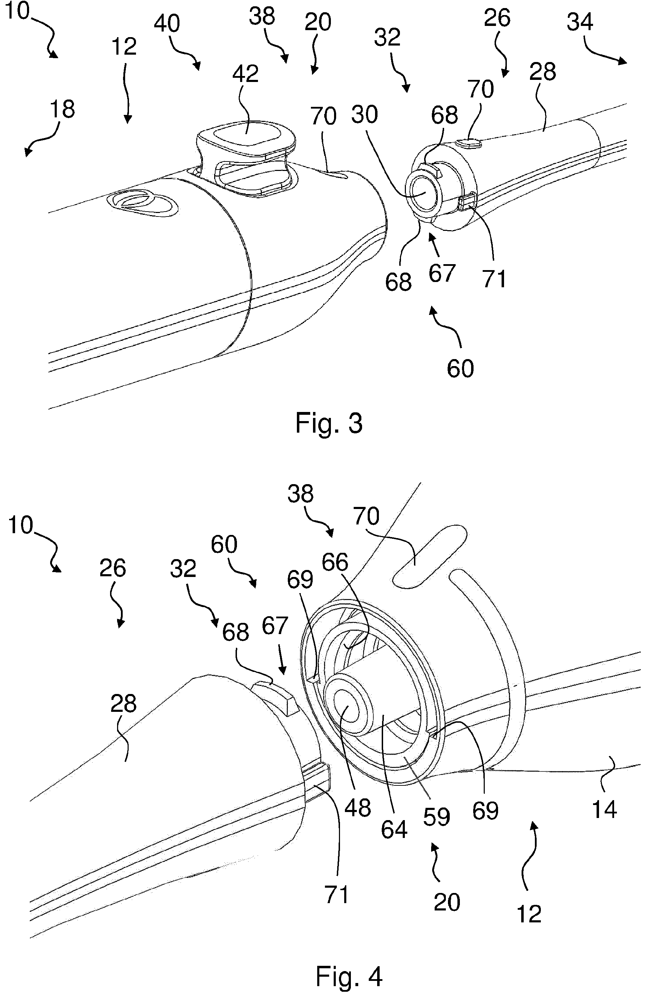

The quick release coupling 60 further comprises two stop surfaces 69 provided on the body member 12 (see Figs. 4 and 5). The stop surfaces 69 may be configured to limit rotation of the body member 12 and/or the rod member 26 relative to each other as the body member 12 and the rod member 26 are being coupled with each other.

The quick release coupling 60 further comprises an engaging section 71 provided on the rod member 26 (see Figs. 3 and 4). The engaging section 71 may be configured to abut at least one of the stop surfaces 69 to limit rotation of the body member 12 and/or the rod member 26 relative to each other as the body member 12 and the rod member 26 are being coupled with each other, the rotation being about the longitudinal axis of the body member 12 and/or the longitudinal axis of the rod member 26.

Thus, as the body member 12 and the rod member 26 are being rotated relative to each other to screw the counter elements 68 into the threaded section 66, the engaging section 71 may come into contact with one of the stop surfaces 69 to limit the rotation of the body member 12 and the rod member 26 relative to each other.

According to the first embodiment shown in Figs. 1 to 5, the two stop surfaces 69 are arranged diametrically opposed from each other. This may allow the rotation of the body member 12 and the rod member 26 relative to each other to be limited to 180°. With respect to the luer lock coupling shown in the first embodiment according to Figs. 1 to 5, screwing the counter elements 68 into the threaded section 66 by a 180° rotation is sufficient to provide a secure, sealed, and stable coupling between the body member 12 and the rod member 26. Limiting the rotation (e.g., after 180° or another angle) may be helpful for avoiding over tightening of the luer connection and/or for avoiding damage to the body member and/or to the rod member by the user.

However, other relative positions between the stop surfaces 69 may also be feasible, e.g., such as to allow a 200° rotation, a 220° rotation, a 240° rotation, a 260° rotation, a 280° rotation or a 300° rotation of the body member 12 relative to the rod member 26.

The engaging section 71 protrudes from a surface of the rod member 26 substantially in a proximal direction, at least when the handheld gynecological device 10 is fully assembled and in use.

Moreover, the engaging section 71 is spaced from the hub 67. The space between the engaging section 71 and the hub 67 may be configured to receive at least a portion of the sleeve section 59 as the body member 12 and the rod member 26 are being coupled with each other. This may help prevent a mistaken assembly by the user.

Thus, as the body member 12 and the rod member 26 are being rotated relative to each other in order to screw the counter elements 68 into the threaded section 66, a portion of the sleeve section 59 is received in the space between the engaging section 71 and the hub 67.

As can be seen in Figs. 1, 2, 4 and 5, the sleeve section 59 may be configured to have varying wall thicknesses and/or varying distance from a wall of the connecting section 38. This may limit the rotational orientation of the rod member 26 relative to the body member 12 to a predetermined range, in which the rod member 26 must be rotationally oriented relative to the body member 12 to allow the body member 12 and the rod member 26 to be coupled with each other via the quick release coupling 60.

For instance, as can be seen in Figs. 1, 2, 4 and 5, the rotational orientation of the rod member 26 relative to the body member 12 is limited to an approximately 180° range. In other words, the rod member 26 must be oriented within this range of rotational orientation relative to the body member 12 in order to allow the body member 12 and the rod member 26 to be coupled with each other via the quick release coupling 60. If the rod member's 26 and the body member's 12 orientation relative to each other is outside of this predetermined range, the sleeve section 59 may not be received in the space between the engaging section 71 and the hub 67, e.g., due to the increased wall thickness of the sleeve section 59 in this range, which is outside of the predetermined range, and/or due to a decreased distance between an outer surface of the sleeve section 59 and a wall of the connecting section 38, which may prevent passage of the engaging section 71 therebetween.

The gynecological device 10 further comprises alignment marks 70. The alignment marks 70 may indicate a starting orientation and/or a final orientation of the rod member 26 and the body member 12 relative to each other prior to and/or upon completion of the coupling of the rod member 26 with the body member 12 via the quick release coupling 60. The alignment marks 70 may thus facilitate the coupling process of the body member 12 with the rod member 26 via the quick release coupling 60.

A first alignment mark 70 is provided on an outwardly facing surface of the rod member 26 and a second alignment mark 70 is provided on an outwardly facing surface of the body member 12. The first alignment mark 70 may be aligned with the second alignment mark 70 in a starting orientation of the rod member 26 relative to the body member 12, i.e., prior to the coupling of rod member 26 with the body member 12 and/or a final orientation of the rod member 26 relative to the body member 12, i.e., upon completion of the coupling of the body member 12 with the rod member 26. Each of the first and/or second alignment mark may be provided as one of a protrusion from the respective outwardly facing surface, a roughened portion on the respective outwardly facing surface, or a printing on the respective outwardly facing surface.

As can be seen in Figs. 1 to 3 and 5, the first alignment mark 70 provided on the rod member 26 may be a protrusion which protrudes beyond an outer surface of the rod member 26.

This may provide a haptic feedback to the user regarding the orientation of the rod member 26 relative to the body member 12. Moreover, configuring the first alignment mark 70 provided on the rod member 26 and/or the second alignment mark 70 provided on the body member as such protrusions may provide a grip assisting feature to facilitate gripping of the rod member 26 and/or the body member 12 when coupling and/or uncoupling the body member 12 and the rod member 26.

**In** order to couple the body member 12 and the rod member 26 with each other, the second tapered section 64 may first be inserted into the first tapered section 62. The alignment marks 70 may indicate a correct starting orientation of the body member 12 relative to the rod member 26.

Subsequently, the counter elements 68 are engaged with the threaded section 66 by rotatably screwing the counter elements 68 into the threaded section 66. As the counter elements 68 are screwed into the threaded section 66, the second tapered section 64 is progressively inserted into the first tapered section 62.

Once the body member 12 and the rod member 26 are snuggly connected to each other, the coupling process is completed.

In order to indicate to the user that the coupling process is completed and/or that the rotational movement of the body member 12 relative to the rod member 26 should be stopped, the alignment marks 70 may be configured to align with each other when the coupling process is completed.

Figs. 6 to 9 show a handheld gynecological device 110 for cervix handling according to a second embodiment of the present invention.

The gynecological device 110 according to the second embodiment differs from the gynecological device 10 according to the first embodiment mainly in the configuration of the quick release coupling, denoted with the reference sign 160 in the embodiment shown in Figs. 6 to 9.

In contrast to the quick release coupling 60 shown in the first embodiment according to Figs. 1 to 5, which is configured as a luer lock coupling, the quick release coupling 160 according to the second embodiment of Figs. 6 to 9 is configured as a bayonet coupling.

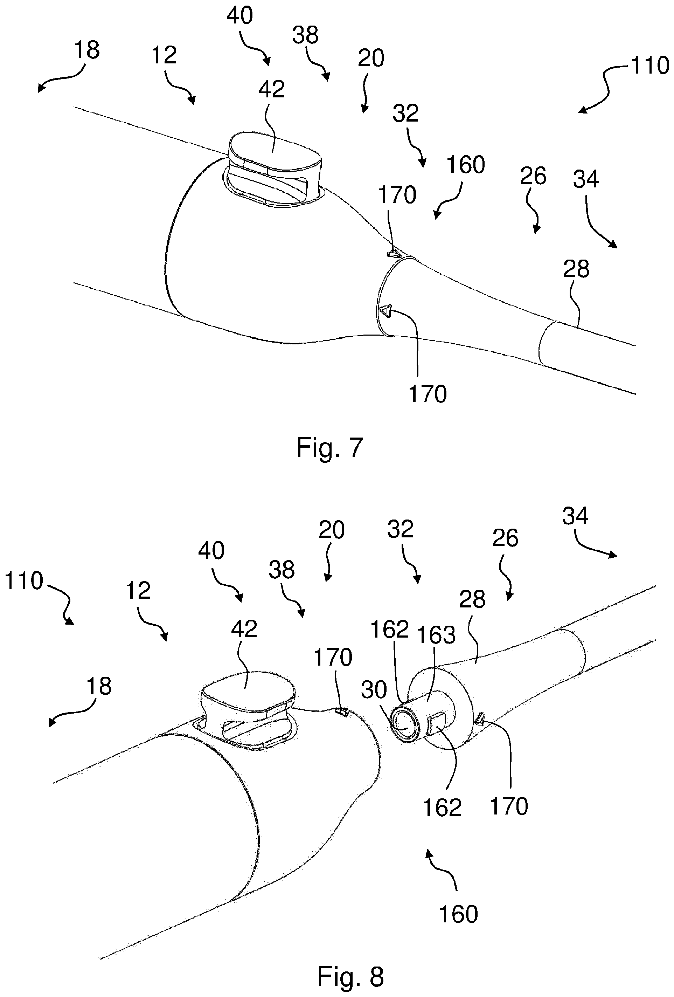

The quick release coupling 160, i.e., configured as a bayonet coupling, comprises two protrusions 162 formed on a surface of a hub 163 of the rod member 26 (see Figs. 6 and 8). According to the second embodiment shown in Figs. 6 to 9, the protrusions 162 extend outwardly from the surface of the hub 163, i.e., away from the longitudinal axis of the rod member 26. However, the protrusions 162 may also extend inwardly from a surface of the rod member 26, i.e., towards the longitudinal axis of the rod member 26.

The quick release coupling 160 further comprises two engaging sections 164 formed on a surface of an inner wall 165 of the body member 12, more specifically of the connecting section 38 (see Fig. 6). According to the second embodiment shown in Figs. 6 to 9, the engaging sections 164 extend inwardly from the surface of the wall 165, i.e., towards the longitudinal axis of the body member 12. However, the engaging sections 164 may also extend outwardly from a surface of the rod member 26, i.e., away from the longitudinal axis of the body member 12.

The engaging sections 164 are spaced from each other circumferentially by two spacing sections 166, only one of which is visible in Fig. 6. In other words, the spacing sections 166 provide openings through which the protrusions 162 can pass as the body member 12 and the rod member 26 are being connected, as described further below.

The protrusions 162 and the engaging sections 164 are configured such that the protrusions 162 can pass the engaging sections 164 as the body member 12 and the rod member 26 are being connected and such that the protrusions 162 can engage the engaging sections 164 after the protrusions 162 have passed the engaging sections 164 to lock the body member 12 and the rod member 26 in position relative to each other.

The gynecological device 110 further comprises alignment marks 170 which are similar, at least in function, to the alignment marks 70 shown in Figs. 1 to 5 with respect to the first embodiment. The alignment marks 170 may indicate a starting orientation and/or a final orientation of the rod member 26 and the body member 12 relative to each other prior to and/or upon completion of the coupling of the rod member 26 with the body member 12 via the quick release coupling 160. The alignment marks 170 may thus facilitate the coupling process of the body member 12 with the rod member 26 via the quick release coupling 160.

A first alignment mark 170 is provided on an outwardly facing surface of the rod member 26 and a second alignment mark 170 is provided on an outwardly facing surface of the body member 12. The first alignment mark 170 may be aligned with the second alignment mark 170 in a starting orientation of the rod member 26 relative to the body member 12, i.e., prior to the coupling of rod member 26 with the body member 12 and/or a final orientation of the rod member 26 relative to the body member 12, i.e., upon completion of the coupling of the body member 12 with the rod member 26.

In order to couple the body member 12 and the rod member 26 with each other, the hub 163 of the rod member 26 may first be inserted into the connecting section 38 of the body member 12. The alignment marks 170 may indicate a correct starting orientation of the body member 12 relative to the rod member 26.

The rod member 26 is oriented relative to the body member 12 such that the protrusions 162 are axially aligned, i.e. in a direction along the longitudinal axis of the body member 12 and/or the longitudinal axis of the rod member 26, with the spacing sections 166. In this orientation, the protrusions 162 can axially, i.e. in a direction along the longitudinal axis of the body member 12 and/or the longitudinal axis of the rod member 26, pass the engaging sections 164 as the hub 163 is being inserted further into the connecting section 38 of the body member 12.

Once the protrusions 162 have passed the engaging sections 164, the rod member 26 may be rotated relative to the body member 12 such that the protrusions 162 axially align, i.e. in a direction along the longitudinal axis of the body member 12 and/or the longitudinal axis of the rod member 26, with at least a section of the engaging sections 164, respectively.

The cooperation between the protrusions 162 and the engaging sections 164 may secure the body member 12 and the rod member 26 together.

Once the body member 12 and the rod member 26 are snuggly connected to each other, the coupling process is completed.

In order to indicate to the user that the coupling process is completed and/or in order to indicate a final rotational position of the body member 12 relative to the rod member 26, the alignment marks 170 may be configured to align with each other when the coupling process is completed and/or when the final relative rotational position has been reached.

Alternatively or additionally, the quick release coupling 160 may comprise at least one stop surface formed or at least provided on a section of one of the body member 12 and/or the rod member 26. A corresponding engaging section may be formed or at least provided on a section of the other of the body member 12 and the rod member 26. The stop surface may cooperate with the engaging section in the coupled state, e.g., to prevent or at least limit rotation of the body member 12 relative to the rod member 26 during the coupling process.

Figs. 10 to 13 show a handheld gynecological device 210 for cervix handling according to a third embodiment of the present invention.

The gynecological device 210 comprises a quick release coupling 260 similar to the quick release coupling 160 of the second embodiment shown in Figs. 6 to 9 in that the quick release coupling 260 is also configured as a bayonet coupling.

In contrast to the quick release coupling 160 of the second embodiment shown in Figs. 6 to 9, the quick release coupling 260 is configured to couple the rod member 26 with a suction head 280.

The suction head 280 comprises an engaging portion 282 configured to engage at least a portion of a cervix of a patient.

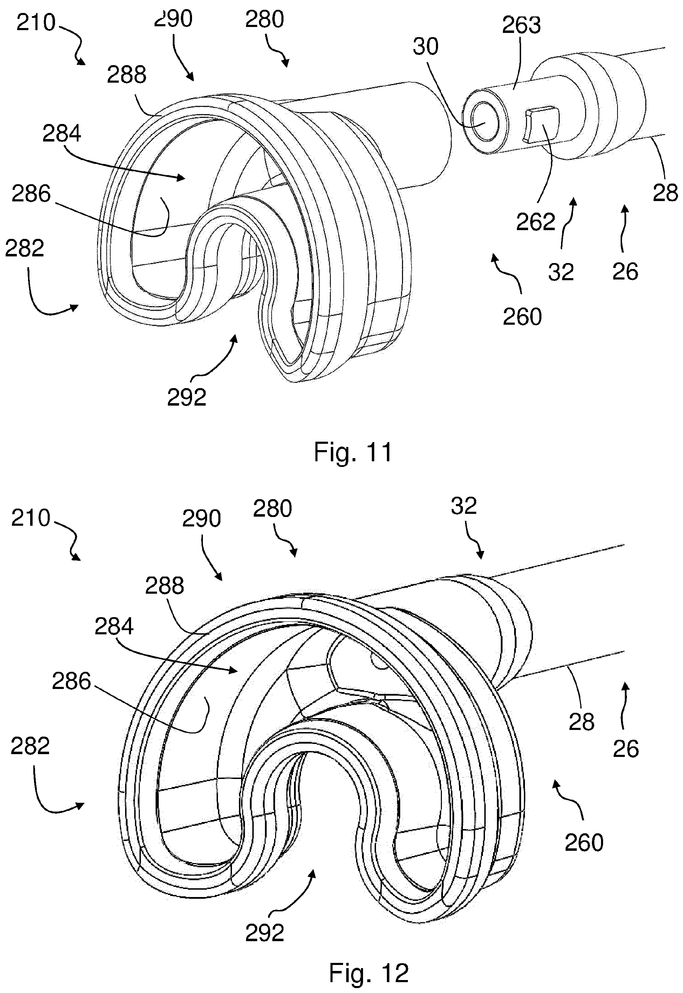

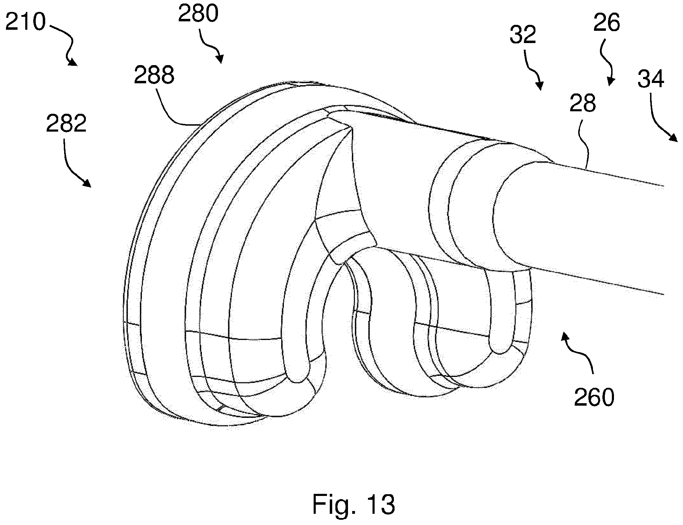

Moreover, the suction head 280 defines a suction chamber 284 fluidly connected to the lumen 30 of the rod member 26 when the rod member 26 is coupled to the suction head 280 (see Figs. 12 and 13).

The suction chamber 284 is delimited by a concave wall 286, the concavity thereof being configured with respect to a distal direction.

An edge 288 of the engaging portion 286 is configured in a C-shape.

The edge 288 forms a closed loop comprising at least a first edge portion 290 and a second edge portion 292. Preferably, the first edge portion 290 and the second edge portion 290 do not lie in a common plane.

A cross-section of the suction chamber 284, the cross-section being substantially perpendicular to the longitudinal axis of the rod member 26, when the rod member 26 is coupled with the suction head 280, is larger than a cross-section of the rod member 26.

Similar to the quick release coupling 160 shown in Figs. 6 to 9 with respect to the second embodiment, the quick release coupling 260 comprises two protrusions 262 formed on a surface of a hub 263 of the rod member 26 (see Figs. 10 and 11).

According to the third embodiment shown in Figs. 10 to 13, the protrusions 262 extend outwardly, i.e., away from the longitudinal axis of the rod member 26. However, the protrusions 262 may alternatively extend inwardly from a surface of the rod member 26, i.e., towards the longitudinal axis of the rod member 26.

The quick release coupling 260 further comprises two engaging sections 264 formed on a surface of an inner wall 265 of the suction head 280 (see Fig. 10). According to the embodiment shown in Figs. 10 to 13, the engaging sections 264 extend inwardly from the surface of the wall 265, i.e., towards the longitudinal axis of the suction head 280. However, the engaging sections 264 may also extend outwardly from a surface of the suction head 280, i.e., away from the longitudinal axis of the suction head 280.

The engaging sections 264 are spaced from each other circumferentially by two spacing sections 266, only one of which is visible in Fig. 10.

The protrusions 262 and the engaging sections 264 are configured such that the protrusions 262 can pass the engaging sections 264 as the rod member 26 and the suction head 280 are being connected and such that the protrusions 262 can engage the engaging sections 264 after the protrusions 262 have passed the engaging sections 264 to lock the rod member 26 and the suction head 280 in position relative to each other.

The gynecological device 210 may also comprise alignment marks similar, in function and/or structural features, to the alignment marks 70 and 170 shown in Figs. 1 to 9, respectively.

In contrast to the engaging sections 164 shown in Figs. 6 to 9 with respect to the second embodiment, the engaging sections 264 have indentation sections 267, which are recessed, preferably substantially proximally, with respect to the other section(s) of the engaging sections 264.

The indentation sections 267 may provide a pit-like recess (or undercut) for the protrusions 262 to rest when the suction head 280 and the rod member 26 are in a coupled state (i.e., to restrict rotation). Such recess or undercut to restrict rotation may also be provided in connection with the embodiment of Figs. 6 to 9.

In order to couple the rod member 26 and the suction head 280 with each other, the hub 263 of the rod member 26 may first be inserted into the suction head 280. Alignment marks may indicate a correct starting orientation of the rod member 26 relative to the suction head 280.

The rod member 26 is oriented relative to the suction head 280 such that the protrusions 262 are axially aligned, i.e. in a direction along the longitudinal axis of the rod member 26, with the spacing sections 266. In this orientation, the protrusions 262 can axially, i.e., in a direction along the longitudinal axis of the rod member 26, pass the engaging sections 264 as the hub 263 is being inserted further into the suction head 280.

Once the protrusions 262 have passed the engaging sections 264, the rod member 26 may be rotated relative to the suction head 280 such that the protrusions 262 axially align, axially, i.e. in a direction along the longitudinal axis of the rod member 26, with at least a section of the engaging sections 264, respectively.

Each protrusion 262 may be inserted at least partially in a respective indentation section 267, to rotatably secure the rod member 26 and the suction head 280 relative to each other to prevent or at least limit rotational movements of the suction head 280 relative to the rod member 26.

In order to enhance this effect, the gynecological apparatus 210 may comprise a biasing feature, such as a spring, to bias the rod member 26 proximally at least when the protrusions 262 are arranged at least partially in the indentation sections 267.

Alternatively, the protrusions 262 and/or at least portions of the engaging sections 264 may be flexible and/or deflectable such that an elastic force may be effected between the protrusions 262 and/or at least portions of the engaging sections 264, when the protrusions 262 are arranged at least partially in the indentation sections 267.

Once the body member 12 and the rod member 26 are snuggly connected to each other, the coupling process is completed.

In order to indicate to the user that the coupling process is completed and/or in order to indicate a final relative rotational position of the rod member 26 relative to the suction head 280, alignment marks may be configured to align with each other when the coupling process is completed and the final relative rotational position has been reached, as described with respect to the first embodiment and the second embodiment of Figs. 1 to 9, respectively.

The quick release couplings 60, 160 according to the first embodiment shown in Figs. 1 to 5 and the second embodiment shown in Figs. 6 to 9, respectively, may be combined with the quick release coupling 260 according to the third embodiment shown in Figs. 10 to 13.

Thus, a handheld gynecological apparatus may be provided in which the rod member 26 is coupled to the suction head 280 via the quick release coupling 260 and in which the rod member 26 is coupled to the body member 12 via the quick release coupling 60 or 160.

Although not explicitly shown in the figures, alternatively, a handheld gynecological apparatus may be provided in which the rod member 26 is coupled to the suction head 280 via the quick release coupling 60.

Alternatively, the quick release coupling, i.e., any of the quick release couplings 60, 160, 260, between the rod member 26 and the body member 12 or the quick release coupling, i.e., any of the quick release couplings 60, 160, 260, between the rod member 26 and the suction head 280 may be omitted.

Instead, the rod member 26 may be coupled to the body member 12 or the suction head 280 via a different coupling mechanism, such as a press fit, welding (e.g., ultrasound welding) and/or an adhesive connection. Further alternatively, the rod member 26 may be integrally formed, e.g., integrally moulded, with the body member 12 or the suction head 280.

However, providing a quick release coupling between the rod member 26 and the body member 12 and between the rod member 26 and the suction head 280, respectively, may further increase the ability of treating patients with varying anatomies.

Hence, the present invention is not limited to the embodiments described and/or shown herein.

## Claims

1. A handheld gynecological apparatus (10; 110; 210) for cervix handling, comprising:
- a body member (12) having a proximal end (18) and a distal end (20);
- a rod member (26) having a proximal end (32) and a distal end (34), the rod member (26) defining a lumen (30) and being configured to be fluidly connected to the distal end (20) of the body member (12);
- a suction head (280) having an engaging portion (282) configured to engage at least a portion of a cervix of a patient, the suction head (280) being fluidly connected to the distal end (34) of the rod member (26) and having a cross-section that is larger than a cross-section of the rod member (26);
- a vacuum generating mechanism configured to generate a vacuum within at least a portion of the gynecological apparatus (10; 110; 210); and
- a quick release coupling (60; 160; 260),
wherein a first member and at least one second member of the gynecological apparatus (10; 110; 210) are connectable to each other by the quick release coupling (60; 160; 260) which is configured for manually coupling and uncoupling the first member and the second member to each other,
wherein the first member is the rod member (26) and the second member is one of the body member (12) and the suction head (280),
wherein the quick release coupling (60; 260) has at least one stop surface (69; 269) configured to limit rotation of the first member and/or the second member relative to each other as the first member and the second member are being coupled with each other, wherein the quick release coupling (60; 160; 260) further comprises an engaging section (71) provided on the rod member (26), and wherein the engaging section (71) is configured to abut at least one of the stop surfaces (69; 269) to limit rotation of the body member (12) and the rod member (26) relative to each other as the body member (12) and the rod member (26) are being coupled with each other; **characterized by** further comprising
- at least two alignment marks (70; 170) each configured to indicate a starting orientation and/or a final orientation of at least one of the components of the handheld gynecological apparatus (10; 110; 210) relative to another component of the handheld gynecological apparatus (10; 110; 210) prior to and/or upon completion of the coupling of the first member with the second member via the quick release coupling (60; 160; 260);
wherein at least one first alignment mark (70; 170) of the at least two alignment marks (70; 170) is provided on the first member
and at least one second alignment mark (70; 170) of the at least two alignment marks (70; 170) is provided on the second member.

2. The handheld gynecological apparatus (10; 110; 210) according to claim 1, wherein the quick release coupling (60; 160; 260) is a luer slip coupling, a luer lock coupling, a bayonet coupling, a threaded coupling or a snap fit coupling.

3. The handheld gynecological apparatus (10) according to claim 2, wherein the luer slip coupling and the luer lock coupling each comprise a first tapered section (62) arranged on the first member and a second tapered section (64) arranged on the second member, wherein the first tapered section (62) and the second tapered section (64) are mated to each other such that the first tapered section (62) and the second tapered section (64) can be pressed together to couple the first member and the second member to each other, wherein preferably the luer lock coupling additionally comprises at least one threaded section (66) arranged on the first or the second member and at least one counter element (68) arranged on the other of the first and the second member, the counter element (68) being configured to be screwed into the threaded section (66) to couple the first member and the second member to each other.

4. The handheld gynecological apparatus (110; 210) according to claim 1 or 2, wherein the quick release coupling (160; 260) has at least one protrusion (162; 262) formed on the first member or the second member and the other of the first member and the second member has at least one engaging section (164; 264), wherein the protrusion (162; 262) and the engaging section (164; 264) are configured such that the protrusion (162; 262) can pass the engaging section (164; 264) as the first member and the second member are being connected and such that the protrusion (162; 262) can engage the engaging section (164; 264) after the protrusion (162; 262) has passed the engaging section (164; 264) to lock the first member and second member in position relative to each other, wherein the engaging section (164; 264) preferably comprises at least one opening (166; 266) through which the protrusion (162; 262) can pass as the first member and the second member are being connected.

5. The handheld gynecological apparatus (110; 210) according to claim 4, wherein the first member and/or the second member is/are configured to be rotated relative to each other once the protrusion (162; 262) has passed the engaging section (164; 264) such that the protrusion (162; 262) can engage the engaging section (164; 264) to lock the first member and the second member in position relative to each other.

6. The handheld gynecological apparatus (110; 210) according to claim 4 or 5, comprising a plurality of protrusions (162; 262), preferably wherein at least two protrusions (162; 262) are arranged diametrically opposite from each other.

7. The handheld gynecological apparatus (10; 110; 210) according to any one of the preceding claims, wherein the quick release coupling (60; 160; 260) is configured to provide a fluid-tight seal between the first member and the second member.

8. The handheld gynaecological apparatus (10; 110; 210) according to any one of the preceding claims, wherein the at least one first alignment (70; 170) mark is aligned with the at least one second alignment mark (70; 170) in a starting orientation and/or a final orientation of at least one of the components of the handheld gynecological apparatus (10; 110; 210) relative to another component of the handheld gynecological apparatus (10; 110; 210) prior to and/or upon completion of the coupling of the first member with the second member via the quick release coupling (60; 160; 260), wherein the at least one first and/or second alignment (70; 170) mark preferably is printed onto the first and second member, respectively.

9. The handheld gynaecological apparatus (10; 110; 210) according to any one of the preceding claims, wherein the at least one first and/or second alignment mark is formed as a recess or projection.

10. The handheld gynaecological apparatus (10; 110; 210) according to any one of the preceding claims, wherein the engaging section (71) protrudes from a surface of the rod member (26) substantially in a proximal direction.

11. The handheld gynecological apparatus (10; 110; 210) according to any one of the preceding claims, wherein the first member is connectable to a third member of the gynecological apparatus (10; 110; 210) by a second quick release coupling (60; 160; 260) configured for manually coupling and uncoupling the first member and the third member to each other, wherein preferably
the third member is the other one of the body member (12) and the suction head (280), and/or
the second quick release coupling (60; 160; 260) is a luer slip coupling, a luer lock coupling, a bayonet coupling, a threaded coupling or a snap fit coupling, wherein the luer slip coupling and the luer lock coupling preferably each comprise a first tapered section (62) arranged on the first member and a second tapered section (64) arranged on the third member, wherein the first tapered section (62) and the second tapered section (64) are mated to each other such that the first tapered section (62) and the second tapered section (64) can be pressed together to couple the first member and the third member to each other, wherein preferably the luer lock coupling additionally comprises at least one threaded section (66) arranged on the first or the third member and at least one counter element (68) arranged on the other of the first and the third member, the counter element (68) being configured to screw into the threaded section (66) to couple the first member and the third member to each other.

12. The handheld gynecological apparatus (10; 110; 210) according to claim 11, wherein the second quick release coupling (160; 260) has at least one protrusion (162; 262) formed on the first member or the third member and the other of the first member and the third member has at least one engaging section (164; 264), wherein the at least one protrusion (162; 262) and the at least one engaging section (164; 264) are configured such that the at least one protrusion (162; 262) can pass the engaging section (164; 264) as the first member and the third member are being connected and such that the at least one protrusion (162; 262) can engage the at least one engaging section (164; 264) after the at least one protrusion (162; 262) has passed the engaging section (164; 264) to lock the first member and third member in position relative to each other, wherein the at least one engaging section (164; 264) preferably comprises at least one opening (166; 266) through which the at least one protrusion (162; 262) can pass as the first member and the third member are being connected, and wherein the first member and/or the third member preferably is/are configured to be rotated relative to each other once the at least one protrusion (162; 262) has passed the respective at least one engaging section (164; 264) such that the at least one protrusion (162; 262) can engage the at least one engaging section (164; 264) to lock the first member and the third member in position relative to each other, and/or the quick release coupling (60; 260) has at least one stop surface (69; 269) configured to limit rotation of the first member and/or the third member relative to each other as the first member and the third member are being coupled with each other, and/or the second quick release coupling (60; 160; 260) is configured to provide a fluid-tight seal between the first member and the third member, wherein the at least one protrusion (162; 262) preferably comprises at least two protrusions (162; 262) being arranged diametrically opposite from each other.

13. The handheld gynecological apparatus (110; 210) according to any one of claims 8 to 12, comprising a plurality of protrusions (162; 262), preferably wherein at least two protrusions (162; 262) of the plurality of protrusions (162; 262) are arranged diametrically opposite from each other.

14. The handheld gynecological apparatus (10; 110; 210) according to any one of the preceding claims, wherein the vacuum generating mechanism is
a vacuum pump driven by a drive mechanism, and/or
a pneumatically driven, electrically driven or manually driven vacuum pump, and/or
at least partially integrated within the body member (12).

15. The handheld gynecological apparatus (10; 110; 210) according to any one of the preceding claims,
wherein the rod member (26) is substantially rigid, and/or
wherein the longitudinal axis of the rod member (26) and the longitudinal axis of the body member (12) coincide with each other, and/or
wherein the rod member (26) and/or the body member (12) are substantially hollow cylindrical, and/or
wherein the gynecological apparatus (10; 110; 210) has a total length extending in the direction of the longitudinal axis of the rod member (26) and wherein the total length is 60 cm or less, more preferably 50 cm or less, more preferably 40 cm or less.

## Patentansprüche

1. Ein handgeführtes gynäkologisches Gerät (10; 110; 210) zur Behandlung der Zervix, umfassend:
- ein Körperelement (12) mit einem proximalen Ende (18) und einem distalen Ende (20);
- ein Stabelement (26) mit einem proximalen Ende (32) und einem distalen Ende (34), wobei das Stabelement (26) ein Lumen (30) definiert und so ausgebildet ist, dass es fluidisch mit dem distalen Ende (20) des Körperelements (12) verbunden ist;
- einen Saugkopf (280) mit einem Eingriffsabschnitt (282), der so ausgebildet ist, dass er mit mindestens einem Abschnitt der Zervix einer Patientin in Eingriff kommt, wobei der Saugkopf (280) fluidisch mit dem distalen Ende (34) des Stabelements (26) verbunden ist und einen Querschnitt aufweist, der größer ist als der Querschnitt des Stabelements (26);
- einen Vakuumerzeugungsmechanismus, der so ausgebildet ist, dass er innerhalb mindestens eines Abschnitts der gynäkologischen Vorrichtung (10; 110; 210) ein Vakuum erzeugt; und
- eine Schnelllösekupplung (60; 160; 260),
wobei ein erstes Element und mindestens ein zweites Element der gynäkologischen Vorrichtung (10; 110; 210) über die Schnelllösekupplung (60; 160; 260) miteinander verbindbar sind, die zum manuellen Verbinden und Trennen des ersten Elements und des zweiten Elements miteinander ausgelegt ist;
wobei das erste Element das Stabelement (26) ist und das zweite Element entweder das Körperelement (12) oder der Saugkopf (280) ist,
wobei die Schnelllösekupplung (60; 260) mindestens eine Anschlagfläche (69; 269) aufweist, die so ausgebildet ist, dass sie die Drehung des ersten Elements und/oder des zweiten Elements relativ zueinander begrenzt, während das erste Element und das zweite Element miteinander verbunden werden, wobei die Schnelllösekupplung (60; 160; 260) ferner einen Eingriffsabschnitt (71) umfasst, der an dem Stangenelement (26) vorgesehen ist, und wobei der Eingriffsabschnitt (71) so ausgebildet ist, dass er an mindestens einer der Anschlagflächen (69; 269) anzulegen, um die Drehung des Körperelements (12) und des Stabelements (26) relativ zueinander zu begrenzen, während das Körperelement (12) und das Stabelement (26) miteinander gekoppelt werden;
**dadurch gekennzeichnet, dass** es weiterhin umfasst
- mindestens zwei Ausrichtungsmarkierungen (70; 170), die jeweils dazu ausgelegt sind, eine Ausgangsausrichtung und/oder eine Endausrichtung mindestens eines der Bauteile des handgeführten gynäkologischen Geräts (10; 110; 210) relativ zu einem anderen Bauteil des handgeführten gynäkologischen Geräts anzuzeigen (10; 110; 210) vor und/oder nach Abschluss der Kopplung des ersten Elements mit dem zweiten Element über die Schnelllösekupplung (60; 160; 260);
wobei mindestens eine erste Ausrichtungsmarkierung (70; 170) der mindestens zwei Ausrichtungsmarkierungen (70; 170) an dem ersten Element und mindestens eine zweite Ausrichtungsmarkierung (70; 170) der mindestens zwei Ausrichtungsmarkierungen (70; 170) an dem zweiten Element vorgesehen ist.

2. Das handgeführte gynäkologische Gerät (10; 110; 210) nach Anspruch 1, wobei die Schnelllösekupplung (60; 160; 260) eine Luer-Slip-Kupplung, eine Luer-Lock-Kupplung, eine Bajonettkupplung, eine Gewindekupplung oder eine Schnappkupplung ist.

3. Das handgeführte gynäkologische Gerät (10) nach Anspruch 2, wobei die Luer-Slip-Kupplung und die Luer-Lock-Kupplung jeweils einen ersten, am ersten Element angeordneten konischen Abschnitt (62) und einen zweiten, am zweiten Element angeordneten konischen Abschnitt (64) umfassen, wobei der erste konische Abschnitt (62) und der zweite konische Abschnitt (64) so aufeinander abgestimmt sind, dass der erste konische Abschnitt (62) und der zweite konische Abschnitt (64) zusammengedrückt werden können, um das erste Element und das zweite Element miteinander zu koppeln, wobei die Luer-Lock-Kupplung vorzugsweise zusätzlich mindestens einen Gewindeabschnitt (66), der an dem ersten oder dem zweiten Element angeordnet ist, und mindestens ein Gegenelement (68) umfasst, das an dem anderen der ersten und zweiten Elemente angeordnet ist, wobei das Gegenelement (68) so ausgebildet ist, dass es in den Gewindeabschnitt (66) eingeschraubt werden kann, um das erste Element und das zweite Element miteinander zu koppeln.

4. Das handgeführte gynäkologische Gerät (110; 210) nach Anspruch 1 oder 2, wobei die Schnelllösekupplung (160; 260) mindestens einen Vorsprung (162; 262) aufweist, der an dem ersten Element oder dem zweiten Element ausgebildet ist, und das jeweils andere der ersten und zweiten Elemente mindestens einen Eingriffsabschnitt (164; 264) aufweist, wobei der Vorsprung (162; 262) und der Eingriffsabschnitt (164; 264) so ausgebildet sind, dass der Vorsprung (162; 262) den Eingriffsabschnitt (164; 264) passieren kann, während das erste Element und das zweite Element miteinander verbunden werden, und dass der Vorsprung (162; 262) in den Eingriffsabschnitt (164; 264) eingreifen kann, nachdem der Vorsprung (162; 262) den Eingriffsabschnitt (164; 264) passiert hat, um das erste Element und das zweite Element relativ zueinander in ihrer Position zu arretieren, wobei der Eingriffsabschnitt (164; 264) vorzugsweise mindestens eine Öffnung (166; 266) aufweist, durch die der Vorsprung (162; 262) hindurchtreten kann, während das erste Element und das zweite Element miteinander verbunden werden.

5. Das handgeführte gynäkologische Gerät (110; 210) nach Anspruch 4, wobei das erste Element und/oder das zweite Element so ausgebildet ist/sind, dass es/sie relativ zueinander gedreht werden kann/können, sobald der Vorsprung (162; 262) den Eingriffsabschnitt (164; 264) passiert hat, so dass der Vorsprung (162; 262) in den Eingriffsabschnitt (164; 264) eingreifen kann, um das erste Element und das zweite Element relativ zueinander in ihrer Position zu arretieren.

6. Das handgeführte gynäkologische Gerät (110; 210) nach Anspruch 4 oder 5, das eine Vielzahl von Vorsprüngen (162; 262) umfasst, wobei vorzugsweise mindestens zwei Vorsprünge (162; 262) einander diametral gegenüberliegend angeordnet sind.

7. Das handgeführte gynäkologische Gerät (10; 110; 210) nach einem der vorhergehenden Ansprüche, wobei die Schnelllösekupplung (60; 160; 260) so ausgebildet ist, dass sie eine flüssigkeitsdichte Abdichtung zwischen dem ersten Element und dem zweiten Element bildet.

8. Das handgeführte gynäkologische Gerät (10; 110; 210) nach einem der vorhergehenden Ansprüche, wobei die mindestens eine erste Ausrichtungsmarkierung (70; 170) in einer Ausgangsausrichtung und/oder einer Endausrichtung mindestens einer der Komponenten des handgeführten gynäkologischen Geräts (10; 110; 210) relativ zu einer anderen Komponente des handgeführten gynäkologischen Geräts (10; 110; 210) vor und/oder bei Abschluss der Kopplung des ersten Elements mit dem zweiten Element über die Schnelllösekupplung (60; 160; 260) ausgerichtet ist, wobei die mindestens eine erste und/oder zweite Ausrichtungsmarkierung (70; 170) vorzugsweise auf das erste bzw. zweite Element aufgedruckt ist.

9. Das handgeführte gynäkologische Gerät (10; 110; 210) nach einem der vorhergehenden Ansprüche, wobei die mindestens eine erste und/oder zweite Ausrichtungsmarkierung als Vertiefung oder Vorsprung ausgebildet ist.

10. Das handgeführte gynäkologische Gerät (10; 110; 210) nach einem der vorhergehenden Ansprüche, wobei der Eingriffsabschnitt (71) von einer Oberfläche des Stabelements (26) im Wesentlichen in proximaler Richtung vorsteht.

11. Das handgeführte gynäkologische Gerät (10; 110; 210) nach einem der vorhergehenden Ansprüche, wobei das erste Element mit einem dritten Element des gynäkologischen Geräts (10; 110; 210) über eine zweite Schnelllösekupplung (60; 160; 260) verbunden werden kann, die zum manuellen Koppeln und Entkoppeln des ersten Elements und des dritten Elements miteinander ausgelegt ist, wobei vorzugsweise
das dritte Element das jeweils andere von Körperelement (12) und Saugkopf (280) ist, und/oder
die zweite Schnelllösekupplung (60; 160; 260) eine Luer-Slip-Kupplung, eine Luer-Lock-Kupplung, eine Bajonettkupplung, eine Gewindekupplung oder eine Schnappkupplung ist, wobei die Luer-Slip-Kupplung und die Luer-Lock-Kupplung vorzugsweise jeweils einen ersten konischen Abschnitt (62), der am ersten Element angeordnet ist, und einen zweiten konischen Abschnitt (64), der am dritten Element angeordnet ist, umfassen, wobei der erste konische Abschnitt (62) und der zweite konische Abschnitt (64) so miteinander in Eingriff stehen, dass der erste konische Abschnitt (62) und der zweite konische Abschnitt (64) zusammengedrückt werden können, um das erste Element und das dritte Element miteinander zu koppeln, wobei die Luer-Lock-Kupplung vorzugsweise zusätzlich mindestens einen Gewindeabschnitt (66), der an dem ersten oder dem dritten Element angeordnet ist, und mindestens ein Gegenelement (68) umfasst, das an dem jeweils anderen der ersten und dritten Elemente angeordnet ist, wobei das Gegenelement (68) so ausgebildet ist, dass es in den Gewindeabschnitt (66) eingeschraubt werden kann, um das erste Element und das dritte Element miteinander zu koppeln.

12. Das handgeführte gynäkologische Gerät (10; 110; 210) nach Anspruch 11, wobei die zweite Schnelllösekupplung (160; 260) mindestens einen Vorsprung (162; 262) aufweist, der an dem ersten Element oder dem dritten Element ausgebildet ist, und das jeweils andere der ersten und dritten Elemente mindestens einen Eingriffsabschnitt (164; 264) aufweist, wobei der mindestens eine Vorsprung (162; 262) und der mindestens eine Eingriffsabschnitt (164; 264) so ausgebildet sind, dass der mindestens eine Vorsprung (162; 262) den Eingriffsabschnitt (164; 264) passieren kann, während das erste Element und das dritte Element miteinander verbunden werden, und dass der mindestens eine Vorsprung (162; 262) in den mindestens einen Eingriffsabschnitt (164; 264) eingreifen kann, nachdem der Vorsprung (162; 262) den Eingriffsabschnitt (164; 264) passiert hat, um das erste Element und das dritte Element relativ zueinander in ihrer Position zu arretieren, wobei der mindestens eine Eingriffsabschnitt (164; 264) vorzugsweise mindestens eine Öffnung (166; 266) aufweist, durch die der mindestens eine Vorsprung (162; 262) hindurchtreten kann, während das erste Element und das dritte Element miteinander verbunden werden, und wobei das erste Element und/oder das dritte Element vorzugsweise so ausgebildet ist/sind, dass sie relativ zueinander gedreht werden können, sobald der mindestens eine Vorsprung (162; 262) den jeweiligen mindestens einen Eingriffsabschnitt (164; 264) passiert hat, so dass der mindestens eine Vorsprung (162; 262) in den mindestens einen Eingriffsabschnitt (164; 264) eingreifen kann, um das erste Element und das dritte Element relativ zueinander in ihrer Position zu verriegeln, und/oder die Schnelllösekupplung (60; 260) weist mindestens eine Anschlagfläche (69; 269) auf, die so ausgebildet ist, dass sie die Drehung des ersten Elements und/oder des dritten Elements relativ zueinander begrenzt, während das erste Element und das dritte Element miteinander verbunden werden, und/oder die zweite Schnelllösekupplung (60; 160; 260) so ausgebildet ist, dass sie eine flüssigkeitsdichte Abdichtung zwischen dem ersten Element und dem dritten Element bereitstellt, wobei der mindestens eine Vorsprung (162; 262) vorzugsweise mindestens zwei Vorsprünge (162; 262) umfasst, die einander diametral gegenüberliegend angeordnet sind.

13. Das handgeführte gynäkologische Gerät (110; 210) gemäß einem der Ansprüche 8 bis 12, umfassend eine Vielzahl von Vorsprüngen (162; 262), wobei vorzugsweise mindestens zwei Vorsprünge (162; 262) der Vielzahl von Vorsprüngen (162; 262) einander diametral gegenüberliegend angeordnet sind.

14. Das handgeführte gynäkologische Gerät (10; 110; 210) nach einem der vorhergehenden Ansprüche, wobei der Vakuumerzeugungsmechanismus
eine durch einen Antriebsmechanismus angetriebene Vakuumpumpe ist, und/oder
eine pneumatisch, elektrisch oder manuell angetriebene Vakuumpumpe ist, und/oder
zumindest teilweise in das Gehäuseelement (12) integriert ist.

15. Das handgeführte gynäkologische Gerät (10; 110; 210) nach einem der vorhergehenden Ansprüche,
wobei das Stabelement (26) im Wesentlichen starr ist, und/oder
wobei die Längsachse des Stabelements (26) und die Längsachse des Körperelements (12) miteinander zusammenfallen, und/oder
wobei das Stabelement (26) und/oder das Körperelement (12) im Wesentlichen hohlzylindrisch sind, und/oder
wobei die gynäkologische Vorrichtung (10; 110; 210) eine Gesamtlänge aufweist, die sich in Richtung der Längsachse des Stabelements (26) erstreckt, und wobei die Gesamtlänge 60 cm oder weniger, vorzugsweise 50 cm oder weniger, noch bevorzugter 40 cm oder weniger beträgt.

## Revendications

1. Appareil gynécologique portatif (10; 110; 210) destiné à la manipulation du col de l'utérus, comprenant :
- un élément de corps (12) comportant une extrémité proximale (18) et une extrémité distale (20);
- un élément de tige (26) comportant une extrémité proximale (32) et une extrémité distale (34), l'élément de tige (26) définissant un lumen (30) et étant configuré pour être relié de manière fluidique à l'extrémité distale (20) de l'élément de corps (12) ;
- une tête d'aspiration (280) comportant une partie d'engagement (282) configurée pour s'engager avec au moins une partie du col de l'utérus d'une patiente, la tête d'aspiration (280) étant reliée de manière fluidique à l'extrémité distale (34) de l'élément de tige (26) et présentant une section transversale supérieure à celle de l'élément de tige (26) ;
- un mécanisme de génération de vide configuré pour générer un vide à l'intérieur d'au moins une partie de l'appareil gynécologique (10; 110; 210) ; et
- un raccord à dégagement rapide (60; 160; 260),
dans lequel un premier élément et au moins un deuxième élément de l'appareil gynécologique (10; 110; 210) peuvent être reliés l'un à l'autre par le raccord rapide (60; 160; 260), qui est conçu pour permettre de relier et de séparer manuellement le premier élément et le deuxième élément l'un de l'autre ;
dans lequel le premier élément est l'élément tige (26) et le deuxième élément est l'un parmi l'élément corps (12) et la tête d'aspiration (280),
dans lequel le raccord à dégagement rapide (60; 260) comporte au moins une surface d'arrêt (69; 269) configurée pour limiter la rotation du premier élément et/ou du deuxième élément l'un par rapport à l'autre lorsque le premier élément et le deuxième élément sont accouplés l'un à l'autre, dans lequel le raccord rapide (60; 160; 260) comprend en outre une section d'engagement (71) prévue sur l'élément de tige (26), et dans lequel la section d'engagement (71) est configurée pour venir en butée contre au moins l'une des surfaces d'arrêt (69; 269) afin de limiter la rotation de l'élément de corps (12) et de l'élément de tige (26) l'un par rapport à l'autre lorsque l'élément de corps (12) et l'élément de tige (26) sont en cours d'accouplement l'un avec l'autre ;
**caractérisé en ce qu'**il comprend en outre
- au moins deux repères d'alignement (70; 170), chacun étant conçu pour indiquer une orientation initiale et/ou une orientation finale d'au moins l'un des composants de l'appareil gynécologique portatif (10; 110; 210) par rapport à un autre composant de l'appareil gynécologique portatif (10; 110; 210) avant et/ou à l'issue de l'accouplement du premier élément avec le deuxième élément via le raccord rapide (60; 160; 260) ;
dans lequel au moins un premier repère d'alignement (70; 170) parmi les au moins deux repères d'alignement (70; 170) est prévu sur le premier élément et au moins un deuxième repère d'alignement (70; 170) parmi les au moins deux repères d'alignement (70; 170) est prévu sur le deuxième élément.

2. Appareil gynécologique portatif (10; 110; 210) selon la revendication 1, dans lequel le raccord à dégagement rapide (60; 160; 260) est un raccord Luer-Slip, un raccord Luer-Lock, un raccord à baïonnette, un raccord fileté ou un raccord à encliquetage.

3. Appareil gynécologique portatif (10) selon la revendication 2, dans lequel le raccord Luer-Slip et le raccord Luer-Lock comprennent chacun une première section conique (62) disposée sur le premier élément et une deuxième section conique (64) disposée sur le deuxième élément, dans lequel la première section conique (62) et la deuxième section conique (64) s'emboîtent l'une dans l'autre de telle sorte que la première section conique (62) et la deuxième section conique (64) puissent être pressées l'une contre l'autre pour accoupler le premier élément et le deuxième élément l'un à l'autre, dans lequel, de préférence, le raccord Luer-Lock comprend en outre au moins une section filetée (66) disposée sur le premier ou le deuxième élément et au moins un contre-élément (68) disposé sur l'autre des premier et deuxième éléments, l'élément de contre-serrage (68) étant configuré pour être vissé dans la section filetée (66) afin de coupler le premier élément et le deuxième élément l'un à l'autre.

4. Appareil gynécologique portatif (110; 210) selon la revendication 1 ou 2, dans lequel le raccord à dégagement rapide (160; 260) comporte au moins une saillie (162 ; 262) formée sur le premier élément ou le deuxième élément, et l'autre des premier et deuxième éléments comporte au moins une section d'engagement (164; 264), dans lequel la saillie (162; 262) et la section d'engagement (164; 264) sont configurées de telle sorte que la saillie (162; 262) puisse passer la section d'engagement (164; 264) lorsque le premier élément et le deuxième élément sont en cours de connexion, et de telle sorte que la saillie (162; 262) puisse s'engager dans la section d'engagement (164; 264) une fois que la saillie (162; 262) a dépassé la section d'engagement (164; 264) afin de verrouiller le premier élément et le deuxième élément en position l'un par rapport à l'autre, dans lequel la section d'engagement (164; 264) comprend de préférence au moins une ouverture (166; 266) à travers laquelle la saillie (162; 262) peut passer pendant que le premier élément et le deuxième élément sont en cours de connexion.

5. Appareil gynécologique portatif (110; 210) selon la revendication 4, dans lequel le premier élément et/ou le deuxième élément sont configurés pour être tournés l'un par rapport à l'autre une fois que la saillie (162; 262) a dépassé la section d'engagement (164; 264), de telle sorte que la saillie (162; 262) puisse s'engager dans la section d'engagement (164; 264) pour verrouiller le premier élément et le deuxième élément en position l'un par rapport à l'autre.

6. Appareil gynécologique portatif (110; 210) selon la revendication 4 ou 5, comprenant une pluralité de saillies (162; 262), de préférence dans lequel au moins deux saillies (162; 262) sont disposées diamétralement opposées l'une à l'autre.

7. Appareil gynécologique portatif (10; 110; 210) selon l'une quelconque des revendications précédentes, dans lequel le raccord à dégagement rapide (60; 160; 260) est configuré pour assurer une étanchéité aux fluides entre le premier élément et le deuxième élément.

8. Appareil gynécologique portatif (10; 110; 210) selon l'une quelconque des revendications précédentes, dans lequel le au moins un premier repère d'alignement (70; 170) est aligné avec le au moins un deuxième repère d'alignement (70; 170) dans une orientation de départ et/ou une orientation finale d'au moins l'un des composants de l'appareil gynécologique portatif (10; 110; 210) par rapport à un autre composant de l'appareil gynécologique portatif (10; 110; 210) avant et/ou à l'issue de l'accouplement du premier élément avec le deuxième élément via le raccord rapide (60; 160; 260), dans lequel le ou les premiers et/ou deuxièmes repères d'alignement (70; 170) sont de préférence imprimés respectivement sur le premier et le deuxième élément.

9. Appareil gynécologique portatif (10; 110; 210) selon l'une quelconque des revendications précédentes, dans lequel le ou les premiers et/ou seconds repères d'alignement sont réalisés sous la forme d'un évidement ou d'une saillie.

10. Appareil gynécologique portatif (10; 110; 210) selon l'une quelconque des revendications précédentes, dans lequel la section d'engagement (71) fait saillie depuis une surface de l'élément tige (26) sensiblement dans une direction proximale.

11. Appareil gynécologique portatif (10; 110; 210) selon l'une quelconque des revendications précédentes, dans lequel le premier élément peut être relié à un troisième élément de l'appareil gynécologique (10; 110; 210) par un deuxième raccord à dégagement rapide (60; 160; 260) configuré pour accoupler et désaccoupler manuellement le premier élément et le troisième élément l'un à l'autre, dans lequel, de préférence,
le troisième élément est l'autre parmi l'élément de corps (12) et la tête d'aspiration (280), et/ou
le deuxième raccord à dégagement rapide (60; 160; 260) est un raccord Luer-Slip, un raccord Luer-Lock, un raccord à baïonnette, un raccord fileté ou un raccord à encliquetage, dans lequel le raccord Luer-Slip et le raccord Luer-Lock comprennent de préférence chacun une première section conique (62) disposée sur le premier élément et une deuxième section conique (64) disposée sur le troisième élément, dans lequel la première section conique (62) et la deuxième section conique (64) s'emboîtent l'une dans l'autre de telle sorte que la première section conique (62) et la deuxième section conique (64) puissent être pressées l'une contre l'autre pour accoupler le premier élément et le troisième élément l'un à l'autre, dans lequel, de préférence, le raccord Luer-Lock comprend en outre au moins une section filetée (66) disposée sur le premier ou le troisième élément et au moins un contre-élément (68) disposé sur l'autre des premier et troisième éléments, le contre-élément (68) étant configuré pour se visser dans la section filetée (66) afin de coupler le premier élément et le troisième élément l'un à l'autre.

12. Appareil gynécologique portatif (10; 110; 210) selon la revendication 11, dans lequel le deuxième raccord à dégagement rapide (160; 260) comporte au moins une saillie (162; 262) formée sur le premier élément ou le troisième élément et l'autre des premier et troisième éléments comporte au moins une section d'engagement (164; 264), dans lequel la au moins une saillie (162; 262) et la au moins une section d'engagement (164; 264) sont configurées de telle sorte que la au moins une saillie (162; 262) puisse passer la section d'engagement (164; 264) lorsque le premier élément et le troisième élément sont en cours de connexion et de telle sorte que la au moins une saillie (162; 262) puisse s'engager dans la au moins une section d'engagement (164; 264) après que la saillie (162; 262) a dépassé la section d'engagement (164; 264) afin de verrouiller le premier élément et le troisième élément en position l'un par rapport à l'autre, dans lequel la au moins une section d'engagement (164; 264) comprend de préférence au moins une ouverture (166; 266) à travers laquelle la au moins une saillie (162; 262) peut passer pendant que le premier élément et le troisième élément sont en cours de connexion, et dans lequel le premier élément et/ou le troisième élément sont de préférence configurés pour être tournés l'un par rapport à l'autre une fois que la au moins une saillie (162; 262) a dépassé la au moins une section d'engagement respective (164; 264) de sorte que la au moins une saillie (162; 262) puisse s'engager dans la au moins une section d'engagement (164; 264) pour verrouiller le premier élément et le troisième élément en position l'un par rapport à l'autre, et/ou le raccord rapide (60; 260) comporte au moins une surface d'arrêt (69; 269) conçue pour limiter la rotation du premier élément et/ou du troisième élément l'un par rapport à l'autre lors de leur accouplement, et/ou le deuxième raccord rapide (60; 160 ; 260) est configuré pour assurer une étanchéité aux fluides entre le premier élément et le troisième élément, dans lequel la au moins une saillie (162; 262) comprend de préférence au moins deux saillies (162; 262) disposées diamétralement opposées l'une à l'autre.

13. Appareil gynécologique portatif (110 210) selon l'une quelconque des revendications 8 à 12, comprenant une pluralité de saillies (162; 262), de préférence dans lequel au moins deux saillies (162; 262) de la pluralité de saillies (162; 262) sont disposées diamétralement opposées l'une à l'autre.

14. Appareil gynécologique portatif (10; 110; 210) selon l'une quelconque des revendications précédentes, dans lequel le mécanisme de génération de vide est
une pompe à vide entraînée par un mécanisme d'entraînement, et/ou
une pompe à vide à entraînement pneumatique, électrique ou manuel, et/ou
au moins partiellement intégrée dans l'élément de corps (12).

15. Appareil gynécologique portatif (10; 110; 210) selon l'une quelconque des revendications précédentes,
dans lequel l'élément en forme de tige (26) est sensiblement rigide, et/ou
dans lequel l'axe longitudinal de l'élément en forme de tige (26) et l'axe longitudinal de l'élément formant corps (12) coïncident l'un avec l'autre, et/ou
dans lequel l'élément tige (26) et/ou l'élément corps (12) sont sensiblement cylindriques creux, et/ou
dans lequel l'appareil gynécologique (10; 110; 210) présente une longueur totale s'étendant dans la direction de l'axe longitudinal de l'élément tige (26) et dans lequel la longueur totale est de 60 cm ou moins, de préférence de 50 cm ou moins, de préférence encore de 40 cm ou moins.
